# EUROPEAN PATENT APPLICATION

(11) **EP 1 526 184 A1**
(43) Date of publication of application: **27.04.2005**
(21) Application number: 03292663.6
(22) Date of filing: 24.10.2003
(51) Int. Cl.: C12N 15/63, A01K 67/027

(54) **Promoter sequences specific for cells of the B lymphoid lineage and pharmaceutical compositions containing the same**

(71) Applicant: INSTITUT NATIONAL DE LA SANTE ET DE LA RECHERCHE MEDICALE (INSERM), 75654 Paris Cédex 13 (FR)
(72) Inventor: Tonelle, Cécile, 13470 Carnoux en Provence (FR); Moreau, Thomas, 13006 Marseille (FR); Chabannon, Christian, 13009 Marseille (FR); Imbert, Jean, 13011 Marseille (FR)
(74) Representative: Grosset-Fournier, Chantal Catherine

(57) **Abstract**

The present invention relates to a nucleic acid sequence comprising or consisting of a S sequence, said S sequence comprising or consisting of:
(1) a first sequence corresponding to a fragment of at least 10 nucleotides of the promoter sequence of the human CD19 gene, such as defined in Figure 1, the ends of which being comprised from the nucleotide at position -1500 to the nucleotide at position -1000 of the promoter sequence of the human CD19 gene,
(2) a second sequence consisting of the contiguous nucleotides ranging from position -231 to position -1 (SEQ ID NO : 1) of the promoter sequence of the human CD19 gene.

## Description

The present invention particularly relates to B lymphoid lineage specific promoter sequences and to pharmaceutical compositions containing said sequences.

Gene transfer into hematopoietic stem cells remains a major objective and challenge in medical science, particularly for gene or cell therapy. The field still faces many technological obstacles, including the difficulty to transduce mostly non-dividing cells, the risk of mutational insertion, and the difficulty to control transgene expression. Over the last few years, lentiviruses have been increasingly used and substituted to onco-retroviruses, because of their ability to integrate the genome of quiescent cells, and the reduced need to use exogenous cytokines to induce cell cycling ^{1;2}. Efficient transduction of human hematopoietic progenitor cell populations, including CD34⁺ cells³, CD34⁺/CD38- cells and SCID-repopulating cells (SRC) ⁴ has been documented with lentiviral vectors.

Lentiviruses also offer advantages over onco-retroviruses, in that they allow for the more efficient integration of regulatory sequences that target the transgene expression to a defined lineage. Several examples have been reported, both with onco-retroviral and lentiviral vectors, that limit the expression of a marker gene to one or several lineages with various efficacies. These include the transcriptional targeting of T-cells^{5; 6}, antigen presenting cells (APC) cells⁷, monocytes/macrophages ⁸ and erythroid cells⁹; ¹⁰.

The possibility to limit transgene expression to the B-cell compartment, following transduction of stem and progenitor cells has rarely been reported so far. In one instance, a sequence from the immunoglobulin heavy chain enhancer in a lentiviral vector was described to increase marker gene expression in B cells ¹⁷. Besides, several reports mentioned the promising use of the human CD19 promoter to target gene expression to B cells in different experimental systems.

The CD19 antigen, under the control of the BSAP (Pax5) transcription factor, is a specific marker of B cells, appears at the pre-B stage, and remains expressed throughout B-cell differentiation and maturation, to be lost at the final stage when B-lymphocytes maturate into plasma cells¹¹. CD19 takes part to the CD21, CD81 and Leu-13 complex which regulates thresholds of intracellular signals¹².

Large regions (6.3 to 4.2kb) that flank the human CD19 gene drove expression of the transgene in the B cell lineage in transgenic mice ^{13; 14; 15}. However, the large fragments of the CD19 promoter described to date are ill-suited for an efficient insertion into a vector, such as a lentiviral vector, because of their size.

In transfection assays, a short region (200bp) was identified in the proximal region of the human CD 19 gene promoter, that produced high levels of reporter activity in lymphoid cell lines¹⁶. But its B lineage-specific promoter activity has never been soundly assessed.

Thus, an object of the present invention is to provide new fragments derived from the CD19 promoter sequence, which are smaller than the large fragments used in transgenic mice and/or more B lineage specific than those of the prior art.

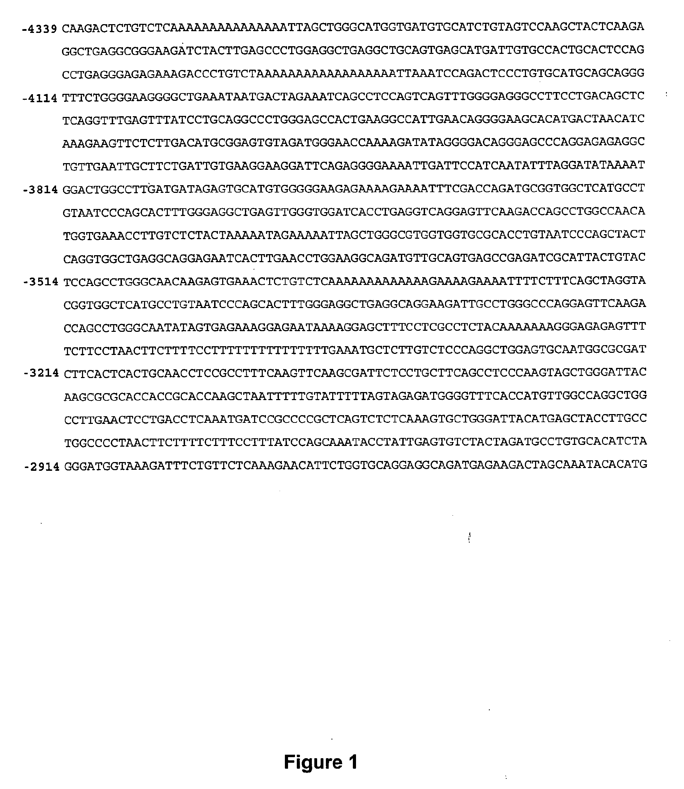

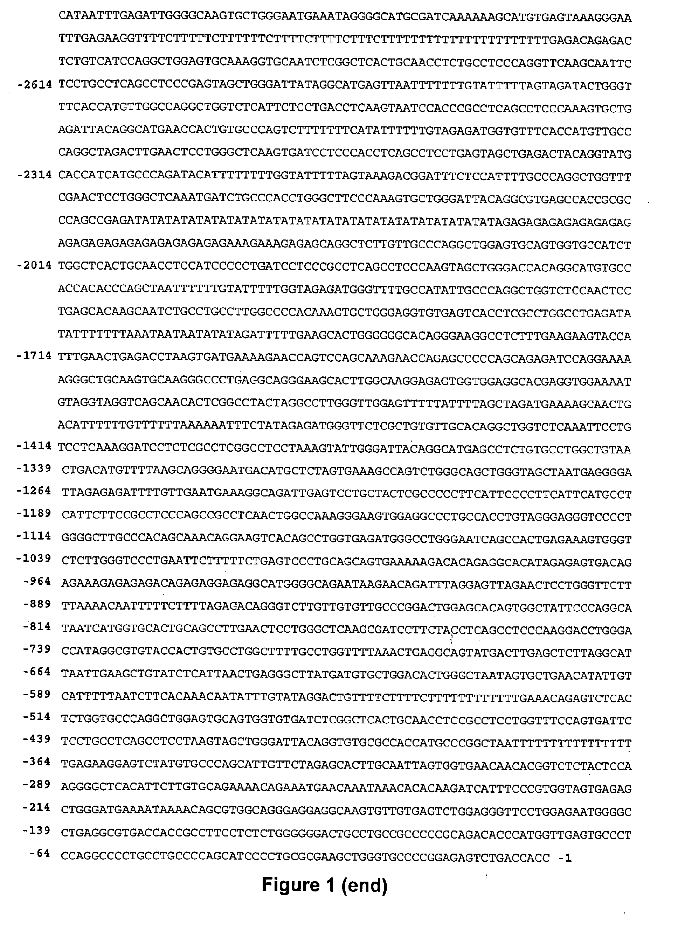

The present invention relates to a nucleic acid sequence comprising or consisting of:
- a S sequence, said S sequence comprising or consisting of:
   (1) a first sequence corresponding to a fragment of at least 10 nucleotides of the promoter sequence of the human CD19 gene, such as defined in Figure 1, the ends of which being comprised from the nucleotide at position -1500 to the nucleotide at position -1000 of the promoter sequence of the human CD19 gene,
   (2) a second sequence consisting of the contiguous nucleotides ranging from position -231 to position -1 (SEQ ID NO :1) of the promoter sequence of the human CD 19 gene; or
- a sequence derived from the above defined S sequence by insertion, deletion or mutation of at least one nucleotide, or presenting an homology of at least 60 % per block of 150 to 200 contiguous nucleotides with the above defined S sequence, provided that said sequence is capable of directing the specific expression of a gene in cells of the B lymphoid lineage;
provided that when said S sequence consists of the contiguous nucleotides of the promoter sequence of the human CD19 gene ranging from the 5' end of the first sequence to position -1, then, if present, the 5' contiguous nucleotides flanking the 5'end of the first sequence in said nucleic acid sequence are different from the 5' contiguous nucleotides flanking the 5' end of the sequence corresponding to said first sequence in the promoter sequence of the human CD 19 gene.

The expression "per block of 150 to 200 contiguous nucleotides" means that the sequence which is homologous to the S sequence comprises at least one fragment of 150 to 200 contiguous nucleotides presenting a homology of at least 60% with the corresponding fragment of the S sequence, provided that said sequence is capable of directing the specific expression of a gene in cells of the B lymphoid lineage.

According to a preferred embodiment, the invention also relates to a nucleic acid sequence as defined above, comprising or consisting of a sequence homologous to the S sequence, said homologous sequence comprising two fragments presenting a homology of at least 73% and 63% respectively with the corresponding fragments of the S sequence, said fragments corresponding respectively to the contiguous nucleotides ranging from position -193 to position -1 (CR1) and from position -1260 to position -1062 (CR2) of the promoter sequence of the human CD19 gene.

In an advantageous embodiment, the invention also relates to a nucleic acid sequence as defined above, comprising or consisting of a sequence presenting an homology of at least 80%, in particular 90% with the above defined S sequence, provided that said sequence is capable of directing the specific expression of a gene in cells of the B lymphoid lineage.

The expression "capable of directing the specific expression of a gene" means that the corresponding sequence can activate the transcription of sequences which are situated on the 3' side of said sequence, and preferably of sequences, the initiation codon of which is linked to the 3' end of said sequence. Such a sequence corresponds in particular to a promoter sequence.

According to a particular embodiment, the S sequence consists of:
(1) a first sequence consisting of the contiguous nucleotides ranging from position X to position Y of the promoter sequence of the human CD19 gene, X and Y being negative integer numbers, and
(2) a second sequence consisting of contiguous nucleotides ranging from position Z to position -1 of the promoter sequence of the human CD19 gene, Z being a negative integer number,
wherein X, Y and Z are such, that:
X ranges from -1500 to -1000,
Y ranges from X+9 to Z-1, and
Z ranges from Y+1 to -231,
the 3' end of said first sequence being covalently linked to the 5' end of said second sequence.

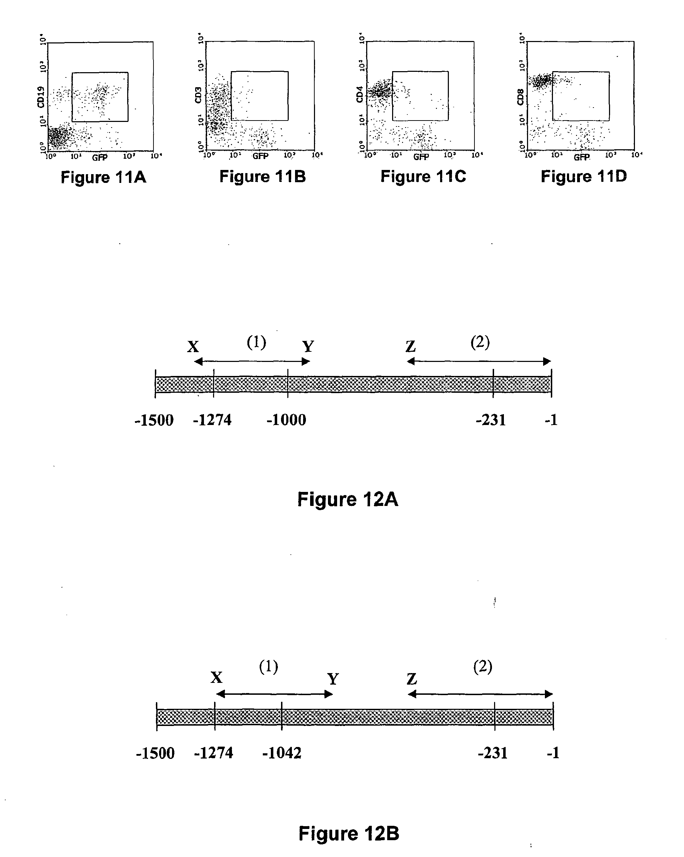

For clarity sake the above defined S sequence is represented in Figure 12A. Preferably the 3' end is linked to the 5' end via a phosphoester linkage.

According to a more particular embodiment,
X=-1274
Y ranges from -1042 to Z-1, and
Z ranges from Y+1 to -231.

For clarity sake the above defined S sequence is represented in Figure 12B.

According to a preferred embodiment,
X = -1274,
Y = -1042, and
Z = -231,
that is, the invention relates to a nucleic acid sequence comprising or consisting of a S sequence, said S sequence consisting of:
(1) a first sequence consisting of the contiguous nucleotides ranging from position -1274 to position -1042 (SEQ ID NO: 2) of the promoter sequence of the human CD19 gene, and
(2) a second sequence consisting of the contiguous nucleotide ranging from position -231 to position -1 (SEQ ID NO: 1) of the promoter sequence of the human CD19 gene,
the 3' end of said first sequence being covalently linked to the 5' end of said second sequence, said S sequence corresponding to SEQ ID NO: 3.

SEQ ID NO: 3 is hereafter referred to as M.CD19.

Advantageously, SEQ ID NO: 3 comprises two elements hereafter referred to as CR1 (position -1 to position -193 of the promoter sequence of the human CD19 gene) and CR2 (position -1062 to position -1260 of the promoter sequence of the human CD19 gene), said elements providing a potent and specific promoter activity in cells of the B lineage.

According to another preferred embodiment,
X is equal to -1274, and
Y = Z-1,
that is, the invention relates to a nucleic acid sequence comprising or consisting of a S sequence, said S sequence consisting of the contiguous nucleotides ranging from position -1274 to position -1 (SEQ ID NO: 4) of the promoter sequence of the human CD19 gene, or of a sequence (SEQ ID NO: 5) derived from said S sequence by the deletion of one nucleotide selected from the contiguous nucleotides ranging from position -541 to position -530 of the promoter sequence of the human CD19 gene, provided that, if present, the 5' contiguous nucleotides flanking the 5'end of the S sequence in said nucleic acid sequence are different from the 5' contiguous nucleotides flanking the nucleotide at position -1274 in the promoter sequence of the human CD 19 gene.

SEQ ID NO: 4 is hereafter referred to as L.CD19.

Advantageously, SEQ ID NO: 4 and SEQ ID NO: 5 comprise two elements hereafter referred to as CR1 (position -1 to position -193 of the promoter sequence of the human CD19 gene) and CR2 (position -1062 to position -1260 of the promoter sequence of the human CD19 gene), said elements providing a potent and specific promoter activity in cells of the B lineage.

The invention also relates to an expression sequence, characterized in that it comprises a nucleic acid sequence such as defined above, linked to a protein encoding nucleic acid sequence, in particular a protein encoding nucleic acid sequence coding for a protein expressed specifically in cells of the B lymphoid lineage, such as a sequence encoding Btk, Igα, surrogate light chains, BLNK, Igb, or to a hairpin RNA encoding nucleic acid sequence, or to a suicide gene such as the HSVTK gene (Herpes simplex virus thymidine kinase), or to a marker gene such as the GFP gene (green fluorescent protein) or the LacZ gene.

Cells of the B lymphoid lineage notably comprise proB cells, preB cells, immature B cells, mature B cells, memory B cells, or cells of the Nalm6, Ramos, and Daudi JEA2 cell lines for example.

The expression "suicide gene" refers to a gene which induces cell death in response to a drug, for cells in which it is expressed. The HSVTK gene thus induces ganciclovir-mediated cell death.

The expression "marker gene" refers to a gene which codes for a protein which is liable to be detected according to methods well known to the man skilled in the art, such as microscopy, in particular fluorescent microscopy. Such a gene notably comprises genes encoding for fluorescent proteins well known to the man skilled in the art, such as GFP and GFP derivatives.

Btk (Bruton tyrosine kinase), Igα, surrogate light chains, BLNK (B cell Linker protein), Igb are notably described in Ballow (2002) *J*. *Allergy Clin. Immunol.* **109:**581, Conley and Cooper (1998) *Curr. Opin. Immunol.* **10:**399 and Gauld *et al.* (2002) *Science* **296:**1641.

Advantageously the abovementioned expression sequences are expressed specifically in cells of the B lymphoid lineage.

According to a preferred embodiment, the abovementioned nucleic acid sequence of said expression sequence consists of SEQ ID NO: 3, 4 or 5.

The invention also relates to a vector, in particular an expression vector, characterized in that it comprises a nucleic acid sequence or an expression sequence such as defined above.

Vectors according to the invention comprise in particular plasmidic vectors such as the pRSET vector or the pcDNA vector, or lentiviral vectors, such as the pTRIP and pHPT vectors.

Advantageously, such vectors are well suited to express RNA and/or proteins in cells of the B lymphoid lineage.

According to a preferred embodiment, the abovementioned nucleic acid sequence of said vector consists of SEQ ID NO: 3, 4 or 5, such as for the lentiviral vectors corresponding respectively to SEQ ID NO: 6, 7 and 8.

The invention also relates to a cell, in particular a cell of the B lymphoid lineage, comprising a nucleic acid sequence such as defined above, or an expression sequence such as defined above, or a vector such as defined above.

Cells according to the invention comprise in particular stem cells, hematopoietic stem cells, embryonic stem cells and precursor cells.

According to a preferred embodiment, the abovementioned cell comprises a nucleic acid sequence consisting of SEQ ID NO: 3, 4 or 5.

The invention also relates to a nonhuman transgenic animal, characterized in that all or some of its cells comprise a nucleic acid sequence such as defined above, or an expression sequence such as defined above, or a vector such as defined above, said animal being in particular a mammal, such as a mouse or a rat.

Such animals can be prepared according to methods well known to the man skilled in the art such as described for example in Maas *et al.* (1999) *J*. *Immunol.* **162**:6526 and Lois *et al.* (2002) *Science* **295**:868.

The invention also relates to a pharmaceutical composition comprising as active substance a nucleic acid sequence such as defined above, or an expression sequence such as defined above, or a vector such as defined above, or a cell such as defined above, in association with a pharmaceutically acceptable vehicle.

The invention also relates to the use of a nucleic acid sequence such as defined above, or an expression sequence such as defined above, or a vector such as defined above, or a cell such as defined above, for the manufacture of a medicament intended to treat pathologies in which cells of the B lymphoid lineage are involved, such as autoimmune diseases, B lymphomas, B leukemias, antibody deficiencies, in particular X linked agammaglobulinemia (Bruton's disease), or diseases resulting from a deficient preB cell receptor complex, such as a Igα defect, a surrogate light chains defect, or a BLNK defect.

Such diseases are notably described in Conley and Cooper (1998) *Current Opinion in immunology* **10:**399-406 ; Gauld *et al.* (2002) *Science* **296:**1641-1642; and Ballow (2002) *J. Allergy Clin. Immunol.* **109:**581-591.

### DESCRIPTION OF THE FIGURES

### Figure 1

Figure 1 represents the upstream nucleic acid sequence of the human CD19 gene. The 5' end corresponds to the nucleotide in position -4339 relative to the A nucleotide of the translation initiation codon (AUG) in position +1, and the 3' end corresponds to the nucleotide in position -1. Nucleotide positions are indicated on the left.

### Figure 2

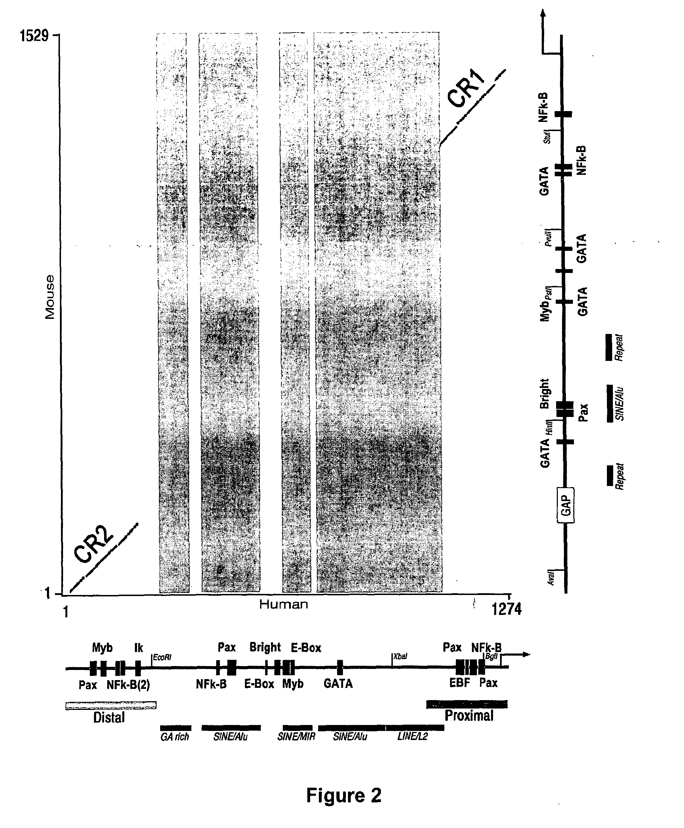

Figure 2 represents the graphical results of the computational analysis of the upstream region of the mouse and human CD 19 genes based on their comparison. The graph abscissae represent the 1274bp of human gene and the ordinates the 1529bp of the murine one. Two conserved regions were identified and schematically drawn: a proximal sequence (CR1) and a distal one (CR2). Putative fixation sites for transcription factors were identified, as indicated by boxes on a horizontal axis for the human gene and on a vertical axis for the murine one. Several restriction sites as well as repetitive sequences (grey bars corresponding to the grey areas on the graph) are also shown.

### Figure 3

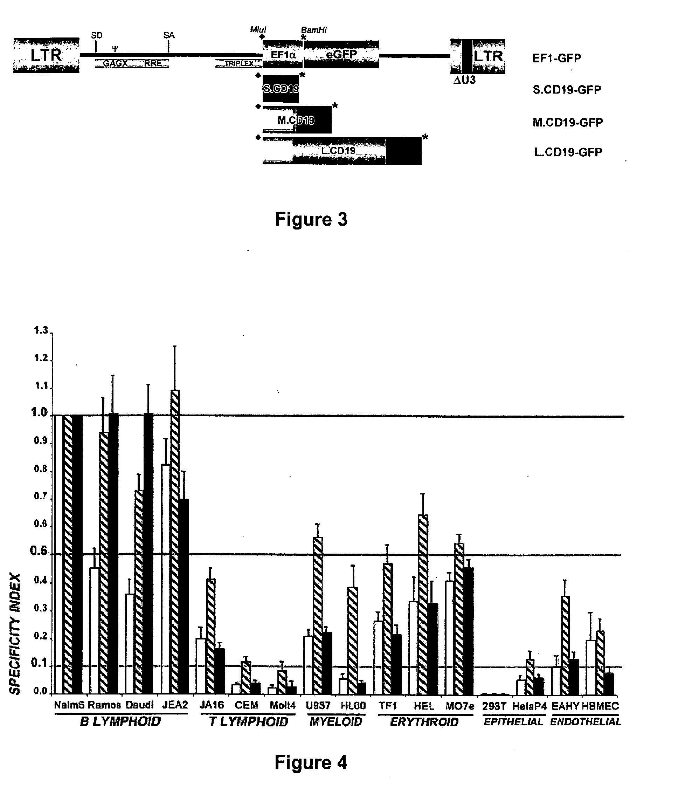

Figure 3 represents the plasmid form of the recombinant lentiviral genomes of the invention. The ubiquitous EF1α promoter (235bp) of pTRIPΔU3-EF1α-GFP vector plasmid was substituted by the S.CD19 (230bp), M.CD19 (464bp) or L.CD19 (1274bp) sequence by cloning between the *Mlu*I (black diamond shape) and *Bam*HI(star) sites. New plasmids were respectively called pTRIPΔU3-S.CD19-GFP, pTRIPΔU3-M.CD19-GFP, pTRIPΔU3-L.CD19-GFP and gave rise to the S.CD19-GFP, M.CD19-GFP and L.CD19-GFP lentiviral vectors. LTR: Long Terminal Repeat; SD/SA: Splice Donor / Splice Acceptor; Ψ: encapsidation signal (on truncated GAG sequence); RRE: Rev Responsive Element; Triplex: Central Termination Sequence (CTS) and central PolyPurine Tract (cPPT) of HIV-1 virus; ΔU3: deletion of 400bp in the 3'LTR U3 region (Self Inactivated vector).

### Figure 4

Figure 4 represents the specificity index (vertical axis) of GFP expression by cell lines (horizontal axis) transduced by the S.CD19-GFP (white bars), M.CD19-GFP (hatched bars) and L.CD19-GFP (black bars) vectors, relative to the GFP expression in the Nalm6 cell line.

### Figures 5A-5I

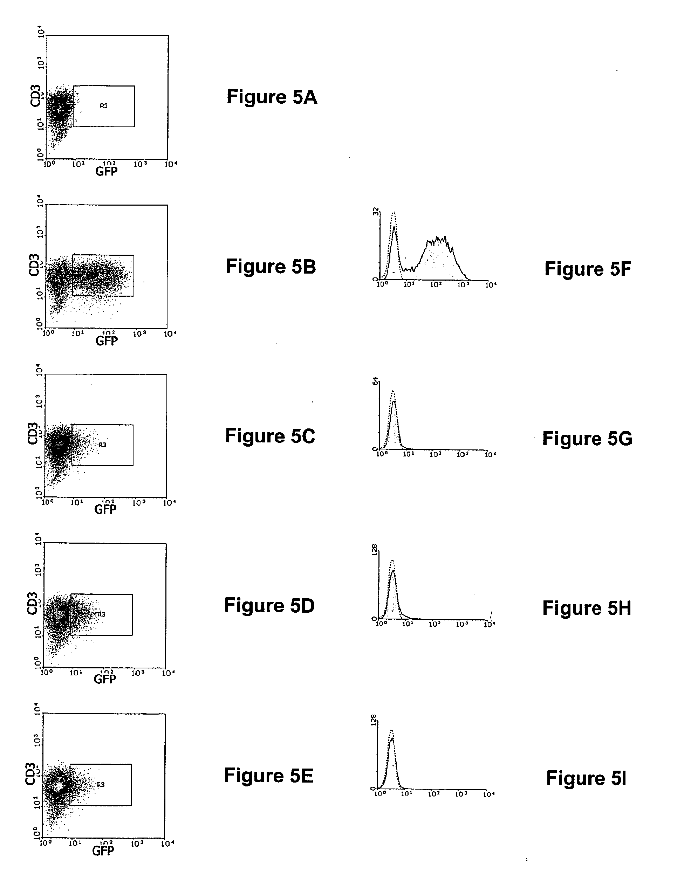

Figures 5A to 5E represent the flow cytometry profile of GFP expression (horizontal axis, fluorescence intensity) by human peripheral T-lymphoid cells, identified by an anti-CD3 antibody (vertical axis, fluorescence intensity), 7 days after transduction in parallel with lentiviral vectors EF1-GFP (Figure 5B), S.CD19-GFP (Figure 5C), M.CD19-GFP (Figure 5D) and L.CD19-GFP (Figure 5E), non-tranduced cells were used as negative control (Figure 5A). The box in the center of each flow cytometry diagram (R3) delimits GFP positive CD3+ cells. Figures 5F to 51 represent the quantitative flow cytometry profile of GFP expression (horizontal axis, fluorescence intensity) by human cord blood CD34+ cells (vertical axis, number of cells), 7 days after transduction in parallel with lentiviral vectors EF1-GFP (Figure 5F), S.CD19-GFP (Figure 5G), M.CD19-GFP (Figure 5H) and L.CD19-GFP (Figure 5I); negative control for GFP expression is shown on each diagram by dotted lines.

### Figures 6A-6T

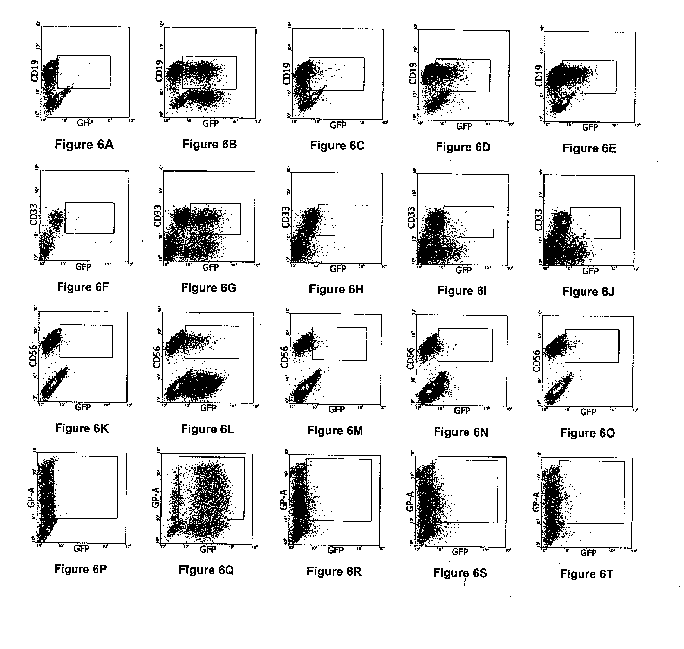

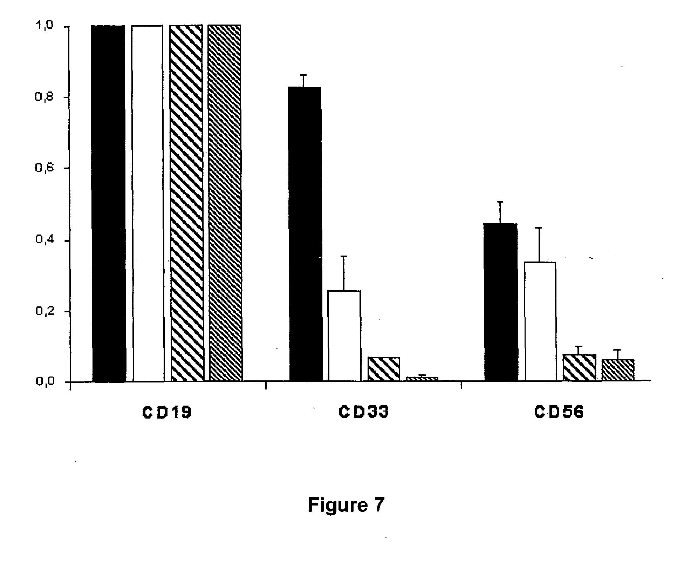

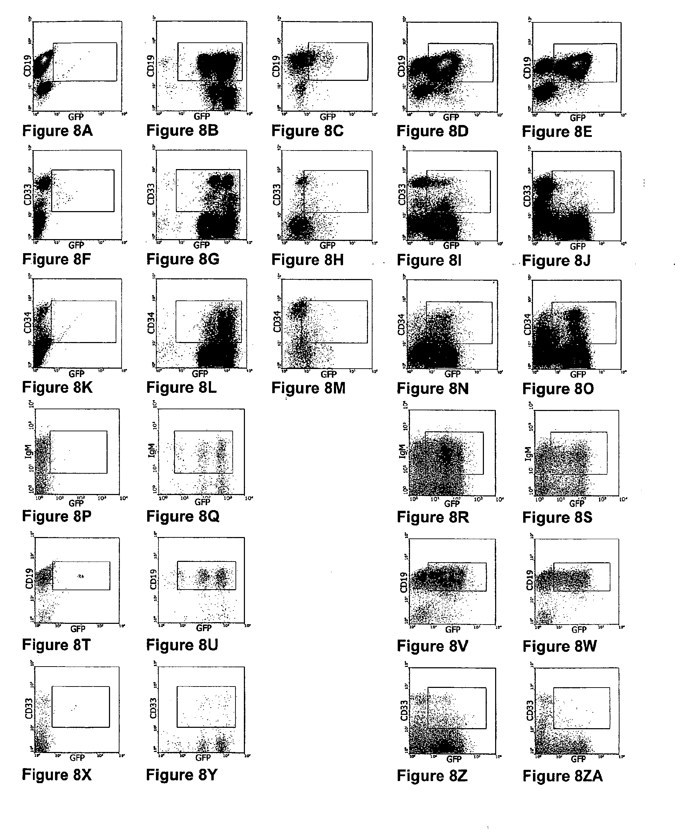

Figures 6A to 6T represent flow cytometry dot blots of GFP expression (horizontal axis, intensity of fluorescence) by cells deriving from transduced and un-transduced CD34+ placental progenitor cells submitted to *in vitro* differentiation by co-culture with the MS.5 cell line (B-lymphoid, myeloid and NK differentiation) or in liquid culture (erythroid differentiation).
Figures 6A to 6E relate to CD19+ cells (vertical axis, intensity of fluorescence), that is B lymphoid cells, deriving from CD34+ progenitor cells untransduced (Figure 6A) or transduced by EF1-GFP (Figure 6B), S.CD19-GFP (Figure 6C), M.CD19-GFP (Figure 6D) and L.CD19-GFP (Figure 6E).
Figures 6F to 6J relate to CD33+ cells (vertical axis, intensity of fluorescence), that is myeloid cells, deriving from CD34+ progenitor cells untransduced (Figure 6F) or transduced by EF1-GFP (Figure 6G), S.CD19-GFP (Figure 6H), M.CD19-GFP (Figure 6I) and L.CD19-GFP (Figure 6J).
Figures 6K to 60 relate to CD56+ cells (vertical axis, intensity of fluorescence), that is NK cells, deriving from CD34+ progenitor cells untransduced (Figure 6K) or transduced by EF1-GFP (Figure 6L), S.CD19-GFP (Figure 6M), M.CD19-GFP (Figure 6N) and L.CD19-GFP (Figure 6O).
Figures 6P to 6T relate to GP-A+ cells (vertical axis, intensity of fluorescence), that is erythroid cells, deriving from CD34+ progenitor cells untransduced (Figure 6P) or transduced by EF1-GFP (Figure 6Q), S.CD19-GFP (Figure 6R), M.CD19-GFP (Figure 6S) and L.CD19-GFP (Figure 6T).
Analyses were done inside the ToPRO-3^{neg}/hCD45+ gate; the box in the center of each flow cytometry diagram delimits lineage+/GFP+ cells.

### Figure 7

Figure 7 represents the specificity index (vertical axis) of the expression of GFP by CD19+, CD33+ and CD56+ cells (horizontal axis) derived *ex vivo* from CD34+ progenitor cells transduced by the EF1-GFP (black bars), S.CD19-GFP (white bars), M.CD19-GFP (largely hatched bars) and L.CD19-GFP (closely hatched bars) vectors, relative to the GFP expression in CD 19+ cells.

### Figures 8A-8ZA

Figure 8A to 8ZA represent flow cytometry diagrams of GFP expression (horizontal axis, intensity of fluorescence) by cells deriving from transduced and un-transduced CD34+ placental progenitor cells submitted to *in vivo* differentiation in the NOD/SCID murine xenograft model. B-lymphoid (CD10, CD19, IgM), myeloid (CD33) and progenitor cells (CD34) compartments were analyzed.
Figures 8A to 8E relate to CD 19+ cells (vertical axis, intensity of fluorescence), taken from bone marrow, deriving from CD34+ progenitor cells untransduced (Figure 8A) or transduced by EF1-GFP (Figure 8B), S.CD19-GFP (Figure 8C), M.CD19-GFP (Figure 8D) and L.CD19-GFP (Figure 8E).
Figures 8F to 8J relate to CD33+ cells (vertical axis, intensity of fluorescence), taken from bone marrow, deriving from CD34+ progenitor cells untransduced (Figure 8F) or transduced by EF1-GFP (Figure 8G), S.CD19-GFP (Figure 8H), M.CD19-GFP (Figure 8I) and L.CD19-GFP (Figure 8J).
Figures 8K to 80 relate to CD34+ cells (vertical axis, intensity of fluorescence), taken from bone marrow, deriving from CD34+ progenitor cells untransduced (Figure 8K) or transduced by EF1-GFP (Figure 8L), S.CD19-GFP (Figure 8M), M.CD19-GFP (Figure 8N) and L.CD19-GFP (Figure 8O).
Figures 8P to 8S relate to IgM+ cells (vertical axis, intensity of fluorescence), taken from the spleen, deriving from CD34+ progenitor cells untransduced (Figure 8P) or transduced by EF1-GFP (Figure 8Q), M.CD19-GFP (Figure 8R) and L.CD19-GFP (Figure 8S).
Figures 8T to 8W relate to CD19+ cells (vertical axis, intensity of fluorescence), taken from the spleen, deriving from CD34+ progenitor cells untransduced (Figure 8T) or transduced by EF1-GFP (Figure 8U), M.CD19-GFP (Figure 8V) and L.CD19-GFP (Figure 8W).
Figures 8X to 8ZA relate to CD33+ cells (vertical axis, intensity of fluorescence), taken from the spleen, deriving from CD34+ progenitor cells untransduced (Figure 8X) or transduced by EF1-GFP (Figure 8Y), M.CD19-GFP (Figure 8Z) and L.CD19-GFP (Figure 8ZA).
Flow cytometry analysis were performed after 12 weeks on bone marrow and spleen gating in ToPRO-3^{neg}/hCD45+ cells; boxes on dot plots delimit lineage+/GFP+ cells

### Figure 9A and Figure 9B

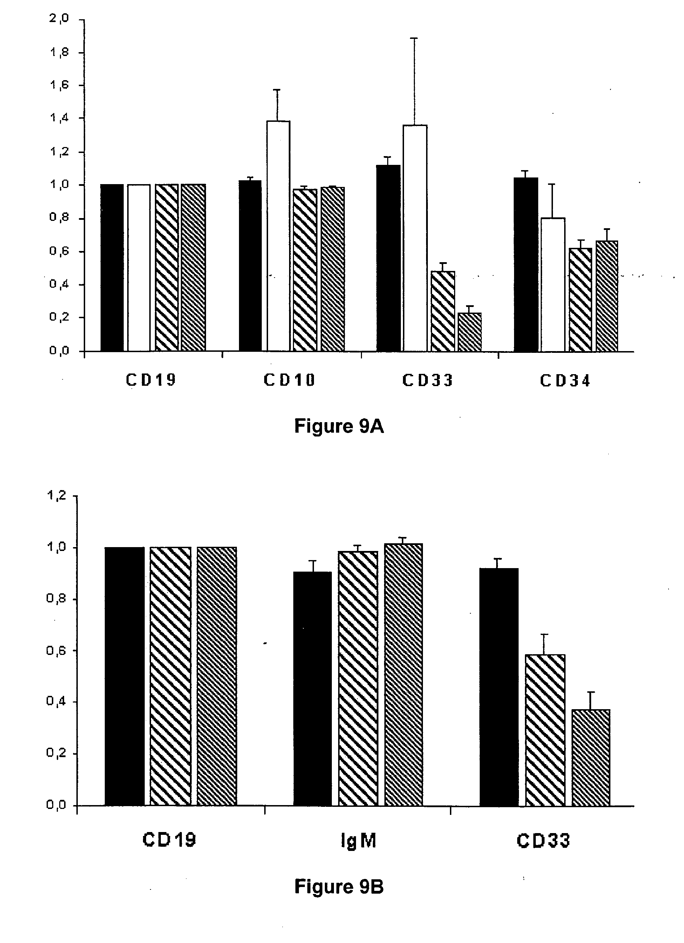

Figure 9A represents the specificity index (vertical axis) of the expression of GFP by CD19+, CD10+, CD33+ and CD34+ bone marrow cells (horizontal axis) *derived in vivo* from CD34+ progenitor cells transduced by the EF1-GFP (black bars), S.CD19-GFP (white bars), M.CD19-GFP (largely hatched bars) and L.CD19-GFP (closely hatched bars) vectors, relative to the GFP expression in CD 19+ cells.
Figure 9B represents the specificity index (vertical axis) of the expression of GFP by CD19+, IgM+ and CD33+ splenic cells (horizontal axis) derived *in vivo* from CD34+ progenitor cells transduced by the EF1-GFP (black bars), M.CD19-GFP (largely hatched bars) and L.CD19-GFP (closely hatched bars) vectors, relative to the GFP expression in CD19+ cells.

### Figure 10A and Figure 10B

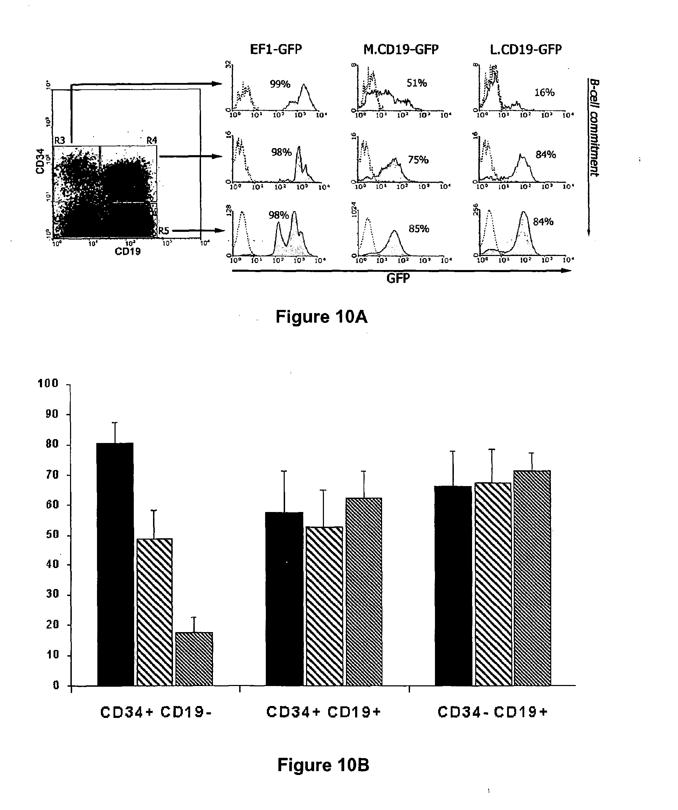

Figure 10A represents the flow cytometry analysis of GFP expression by cells deriving from CD34+ progenitor cells engrafted in NOD/SCID mice.
The flow cytometry dot blot on the left shows a representative experiment of CD34 expression (vertical axis, intensity of fluorescence) and of CD 19 expression (horizontal axis, intensity of fluorescence) of cells taken from mice engrafted with EF1, M.CD19 or L.CD19-GFP transduced CD34+ progenitor cells. The boxes delimit non-B progenitors (CD34+CD19-) (R3), B-progenitors (CD34+CD19+) (R4) and more mature B-cells (CD34-CD19+) (R5).
The histograms on the right represent the GFP expression profile (horizontal axis, fluorescence intensity) corresponding to the abovementioned CD34+CD19- cells (upper histograms), CD34+CD19+ cells (middle histograms) and CD34-CD19+ cells (lower histograms) deriving from CD34+ progenitor cells transduced by EF1-GFP, M.CD19-GFP, L.CD19-GFP.
The percentage of GFP expressing cells is indicated in bold for each histogram, background levels are indicated by dotted lines.
From top to bottom the histograms are representative of a more advanced B cell commitment stage.
Figure 10B shows a histogram representing the percentage of mean GFP expression ± SEM of the CD34+CD19- cells, CD34+CD19+ cells and CD34-CD19+ cells described in figure 10A and deriving from CD34+ progenitor cells transduced by EF1-GFP (black bars), M.CD19-GFP (largely hatched bars) and L.CD19-GFP (closely hatched bars).

### Figure 11A, Figure 11B, Figure 11C and Figure 11D

Figures 11A to 11D represent the flow cytometry expression profile of GFP expression (horizontal axis, fluorescence intensity) by human CD19+ (vertical axis, fluorescence intensity) (Figure 11A), CD3+ (vertical axis, fluorescence intensity) (Figure 11B), CD4+ (vertical axis, fluorescence intensity) (Figure 11C) and CD8+ (vertical axis, fluorescence intensity) (Figure 11D) thymic cells issued from NOD/SCID mouse engrafted by M.CD19-GFP transduced CD34+ cells.
Flow cytometry analysis was performed after 12 weeks, gating in ToPRO-3^{neg}/hCD45+ cells; the box in the center of each flow cytometry diagram delimits lineage+/GFP+ cells.

### Figure 12A and Figure 12B

Figure 12A and 12B represent fragments (arrows) of the 1500 nucleotides upstream of the translation initiation codon of the human CD19 gene (grey box) as defined by their nucleotide positions.
Figure 12A represents the two sequences (1 and 2) constituting the S sequence, wherein X is comprised from positions -1500 to -1000, Y is such that the length of sequence (i) is at least 10 nucleotide long and Z is comprised from positions Y+1 to -231.
Figure 12B represents the two sequences (i and ii) constituting the S sequence, wherein X is equal to -1274, Y is greater or equal to -1042 and Z is comprised from positions Y+1 to -231.

### EXAMPLES

### EXAMPLE 1

### Design of CD1 9-GFP lentiviral vectors and GFP expression in B cell lines transduced by said vectors

### 1. Selection of CD19 B-cell specific regulatory sequences

In order to determine the minimal sequences that are functionally important, and may thus confer B-cell specificity when inserted in a lentiviral vector, a computational sequence analysis approach³⁰ was used to study the CD19 gene regulatory region. Contigs containing the human and mouse CD19 genes were first obtained from the Ensembl genome database (Sanger Institute/EMBL-EBI) (www.ensembl.org)¹⁹.

First, using the RepeatMasker software (available at http://ftp.genome.washington.edu/RM/RepeatMasker.html) (Smit, AFA & Green, P RepeatMasker), repetitive sequences were identified and masked in the human contig AC 109460 containing the CD 19 gene.

Second, comparison with the mouse contig AC125169 was carried out, using the Pipmaker software (Schwartz *et al.* (2000) *Genome Research* **10**:577-586); two conserved regions were identified in the human and mouse CD19 gene promoters ³¹, including a previously non identified 5' upstream fragment.

The two conserved regions, hereafter named CR1 and CR2, were contained in a contiguous 1274 bp fragment of the human CD19 gene **(Figure 2)**. CR2 (198 bp) was located -1 Kb upstream of human CD19 gene initiation site, and at a not yet localized upstream position in the mouse gene, since the sequence of contig AC 125169 is not yet entirely known. To allow for a more precise comparison using Pipmaker, a 1529 bp fragment for the mouse CD19 gene was created, where CR2 was inserted 5' upstream and artificially separated with 100 Ns (undefined nucleotides) from the sequenced mouse CD19 promoter. The CD19 promoter contains several well characterized regulatory elements required for B-cell specific expression such as the binding sites for the B-cell specific transcription factor BSAP/Pax5 ^{16;} ³¹⁻³³ . Both human and mouse CD19 upstream regions were further screened for putative regulatory elements using the TESS software (TESS: Transcription Element Search Software available at http://www.cbil.upenn.edu/tess, Jonathan Schug and G. Christian Overton, Technical Report CBIL-TR-1997-1001-v0.0 Computational Biology and Informatics Laboratory School of Medicine University of Pennsylvania, 1997) and the Matlnspector software²⁰ (available at Genomatix: http://www.genomatix.de)²¹ **(Figure 2).**

Based upon this computational approach, it was hypothesized that the two conserved regions CR1 and CR2, which present respectively 73% and 63% of homology with the corresponding mouse nucleotide sequence, might be functionally important to promote CD19 expression. All CD19 sequences were produced by PCR methods from human primary cell genomic DNA.

Briefly, three sequences based on the sequence of the CD 19 promoter were generated byPCR (S.CD19, M.CD19 and L.CD19).

The following oligonucleotides were used as primers: S.CD19(+) ^{5'}GTT ACG CGT CCC GTG GTA GTG AGA (SEQ ID NO: 9) and S.CD19(-) ^{5'}TTG GGA TCC GGT GGT CAG ACT CTC (SEQ ID NO: 10) for the S.CD19 sequence; L.CD19(+) ^{5'}GTT ACG CGT AAT GAG GGG ATT AGA GAG (SEQ ID NO: 11) and L.CD19(-) ^{5'}TTG GGA TCC GGT GGT CAG ACT CTC CG (SEQ ID NO: 12) for the L.CD19 sequence (underlined sequences are restriction sites for *Ml*uI and *Bam*HI). The generation of the M.CD19 sequence required two PCR steps, in order to juxtapose two distant regions of the native genome; first, two PCR reactions were carried out with the following two primer pairs: L.CD19(+) and M.CD19(-) ^{5'}GCT CTC ACT ACC ACG GGA CCA CTT TCT CAG TGG CTG (SEQ ID NO: 13); M.CD19(+) ^{5'}CAG CCA CTG AGA AAG TGG TCC CGT GGT AGT GAG AGC (SEQ ID NO: 14) and L.CD19(-); purified products were then joined end to end in a single PCR reaction carried out with the L.CD19(+) and L.CD19(-) primer pair. Amplifications were realized in presence of *Platinum HIFI Taq polymerase* (Invitrogen), with the following program: 2' at 94°C followed by 10 cycles with 45s at 94°C, 45s at 58°C and 2' at 68°C, then 25 cycles with 45s at 94°C, 45s at 66°C and 2' at 68°C; the last cycle ended with an extension phase of 7'.

The S.CD19 sequence contains the proximal region of homology (CR1) for a total length of 230bp. The composite M.CD19 (SEQ ID NO: 3) sequence of 464bp length results from the artificial juxtaposition of the two fragments containing the proximal and distal highlighted homologous regions (CR1 and CR2). The last sequence named L.CD19 (SEQ ID NO: 4) corresponds to the entire native CD19 sequence from the initiation site to the CR2 region, and is 1274bp long.

The inventors then designed three new lentiviral vectors, in which the GFP gene was placed under the control of human CD 19 gene promoter fragments.

### 2. Design of CD19-GFP lentiviral vectors

In order to produce B-specific lentiviral vectors, the inventors cloned the three abovementioned CD19 sequences upstream a GFP marker gene between *Mlu*I and *Bam*HI restriction sites of the pTRIPΔU3-EF1-GFP plasmid in place of the ubiquitous EFIα promoter (235bp): the subsequent recombinant lentiviral vectors were respectively called S.CD19-GFP, M.CD19-GFP and L.CD19-GFP **(Figure 3).**

Briefly, the S.CD19, M.CD19 and L.CD19 sequences were introduced in the pTRIPΔU3-EF1α-GFP vector plasmid (*N.B.* all lentiviral constructions were derived from the initial vector backbone pTRIPΔU3-EF1α-GFP [gift from Dr P. Charneau, Institut Pasteur, Paris, France³]), in substitution to the EF1α ubiquitous promoter between *Mlu*I and *Bam*HI sites to obtain the pTRIPΔU3-S.CD19-GFP, the pTRIPΔU3-M.CD19-GFP and the pTRIPΔU3-L.CD19-GFP vector plasmids respectively **(Figure 3).**

Each construction was sequenced in the promoter region to confirm the identity of the CD19 sequence fragment with the genomic CD19 sequence (Genome express, Meylan, France); of note, a single T deletion was observed in a thymidine-rich region (12 repetitions, positions -541 to -530) in the L.CD19 sequence used (SEQ ID NO: 5).

The sequences of the lentiviral vectors comprising the M.CD19, L.CD19 and the deleted L.CD19 promoters, without the GFP gene, are respectively presented in SEQ ID NO: 6, 7 and 8.

Lentiviral particles were then produced by transient co-transfection of three plasmids into 293T cells: the plasmid containing the recombinant vector sequence, the packaging plasmid (p8.91), and the plasmid coding for the VSV-G (Vesicular Stomatitis Virus) envelop protein (pMDG), that confers amphotropic properties to recombinant lentiviral vectors; transfection was done by calcium phosphate co-precipitation, using the Calphos Mammalian transfection kit (Clontech laboratories, Ozyme, Montigny le Bretonneux, France). Supernatants were collected 24, 48 and 72 hours post-transfection, 0.45µm filtered, treated with DNAseI (1µg/mL, Roche Applied Science, Meylan, France) in the presence of MgCl₂ (1nM) for 15'at 37°C and stored at -80°C; samples were tested for the absence of replication competent lentivirus (RCL) by monitoring p24 and Tat protein production in transduced HelaP4 cell cultures²². Finally, lentiviral vector productions were concentrated by ultracentrifugation, aliquoted in serum-free BIT medium (Stem Cell Technologies, Meylan, France) and stored at -80°C until use. Titers of different vectors usually ranged from 1x10⁸ and 1x10⁹ TU/mL (Transduction Unit), as measured by GFP expression on day 7 in the human pre-B cell line Nalm6, transduced in triplicate with serial dilution of concentrated lentiviruses.

### 3. GFP expression in B cell lines transduced with CD19-GFP vectors

The promoter activity of the three CD19 sequences, in the context of lentiviral vectors, was first tested on a panel of hematopoietic and non-hematopoietic human cell lines, comprising:
- human hematopoietic cell lines: B-lymphoid cells (Nalm6, Ramos, Daudi and JEA2), T-lymphoid cells (Jurkat or JA16, CEM and Molt4), myeloid cells (U937, HL60) and erythroleukemic cells (HEL, MO7e, TF1), which were all cultured in RPMI (Biowhittaker, Emerainville, France), 10% FCS (Foetal Calf Serum) (Gibco/Invitrogen), in the presence of antibiotics: 1%PS (100 U/mL Penicillin and 100 U/mL streptomycin, Biowhittaker). For TF1 and MO7e cell lines, culture medium was supplemented by rhGM-CSF (10 ng/mL, Peproteck/Tebu, Le Perray-en Yvelines, France) and rhGM-CSF (20 ng/mL) plus rhSCF (20 ng/mL, Amgen, Thousand Oaks, CA, USA) respectively.
- epithelial cell lines (293T, HelaP4), which were cultured in DMEM (Biowhittaker) 10% FCS, 1% PS; and
- endothelial cell lines (EAHY, HBMEC) in DMEM 20% FCS, 1% PS and DMEM/1% Glucose, 5% FCS, 1% PS, folic acid (1µg/mL), HEPES (10mM), glutamine (3mM) respectively.

All cells were transduced in parallel with the three different CD 19-GFP vectors and the EF1-GFP vector as positive control; we used a MOI between 0.5 and 0.7 that allowed 20 to 50% of positive control cells to express GFP for all cell types.

Cell lines were transduced without stimulation in their initial medium at a cell density of 0.05 to 0.3x10⁶ cells per well (24 well plate) in a total volume of 0.5 mL of their respective culture medium, and in the presence of polybren (4 µg/mL, Sigma Saint Quentin Fallavier, France). Cells were maintained at 37°C for 24 hours before washing with PBS.

Transgene expression was measured by flow cytometry 7 days post-transduction, and the expression specificity index (SI) for CD19 sequences calculated and normalized for each experiments by values obtained in parallel with the pre-B cell line Nalm6.

Briefly, for experiments on cell lines, where EF1-GFP positive control vector was systematically used in parallel of CD19-GFP vectors, the SI was calculated for each cell line as followed: SI= (%GFP+) ^{CD19 vector}/(%GFP+)^{EF1 vector} ; then, individual SI values were normalized by SI value obtained in parallel with the Nalm6 pre-B cell line. For *in vitro* and *in vivo* differentiation experiments, SI was calculated for each lineage as followed: SI=(%GFP+)^{lineage}/(%GFP+)^{CD19+lineage} **(Figure 4)**.

For the present and subsequent cytometry flow analyses, the antibodies used in were murine mAb IgG₁-PC5 (Phycoerythrocyanin 5), IgG₁-PE (Phycoerythrin), hCD45-PC5, hCD3-PE, hCD4-PE, hCD8-PE, hCD10-PE, hCD19-PE, hCD33-PE, hCD34-PE, hCD34-APC (Allophycocyanin) (Beckman Coulter/Immunotech, Marseille, France), hCD56-PE, hIgM-PE and GP-A-PE (BD/Bioscience, Le Pont de Claix, France). Cells were analyzed with a FACScalibur (Becton-Dickinson Immunocytometry systems, BDIS, San Jose, CA, USA). Dead cells were excluded for further analysis, by gating out TO-PRO-3 iodide positive cells at 630 nm, in 0.5 µM concentration (Molecular Probes, Interchim, Montluçon, France).

First, it appeared that CD19 sequences actually showed promoter activity in this lentiviral context. Second, GFP expression driven by any of the CD19 sequences was found preferentially in cell lines of B-lymphoid origin that express CD19 (Nalm6, Ramos, Daudi, JEA2). More precisely, the M and L.CD19 promoters presented a strong activity in all four B-cell lines (0.7>SI>1.09), whereas S.CD19 one appeared more variable (0.35>SI>1). Intensity of GFP expression was quite similar for all CD19 sequences (MFI=20-30), although slightly higher with the M and L.CD19 ones, and still weak in comparison with positive control cells (MFI ~ 100); of note, the MFI varied significantly between experiments, for CD19 promoters as for EF1 promoter. Considering GFP expression in non-B cell lines, experiments showed that the S and L.CD19 sequences had very low promoter activity (0>IS>0.22) in T-lymphoid (JA16, CEM, Molt4), myeloid (U937, HL60), epithelial (293T, HelaP4) and endothelial (EAHY, HBMEC) cells; these two sequences were slightly more active (0.22>IS>0.45) in erythroleukemic cells (TF1, HEL, MO7e). Analysis of the M.CD19 promoter behavior showed a more pronounced aspecific promoter activity in non-B cell lines (0>IS>0.64), especially in myeloid and erythroleukemic cells. To summarize, the L.CD19 sequence presented a more marked B-specific promoter activity than the S or M.CD19 ones that showed respectively low promoter activity in CD19+ B-cell lines and a quite high nonspecific activity in non-B-lymphoid cells.

### 4. Transduction efficiency of the native and CD19 lentiviral vectors .

PCR experiments on genomic DNA of primary human T-lymphoid and progenitor cells revealed a similar integration of the viral genome after transduction with S, M or L.CD19-GFP vectors as compared to EF1-GFP.

Briefly, PCR reactions were performed on 75 ng of purified genomic DNA (Nucleospin Tissue, Macherey Nagel, France) isolated 7 to 14 days after transduction.

Transduction efficiency was measured with real time quantitative PCR, using TaqMan Master mix with the TaqMan® apparatus (Perkin Elmer Applied Biosystems, Courtaboeuf, France). GFP specific pair of primers (Genset) and fluorogenic probe (Perkin-Elmer Applied Biosystems) were designed using Primer Express® software (Perkin-Elmer): GFP-F ^{5'}AGC AAA GAC CCC AAC GAG AA (SEQ ID NO: 15) and GFP-R ^{5'}GGC GGC GGT CAC GAA C (SEQ ID NO: 16) were used at 650nM and the probe CGCGATCACATGGTCCTGCTGG (SEQ ID NO: 17) was at 200nM in a final reaction volume of 25µL. Thermal cycling conditions have been adjusted to the following parameters: 2 min at 50°C and 10 min at 95°C, then 40 cycles of 1 min at 95°C and 1 min at 66°C. In order to determine the lentiviral sequence copy number in each sample, raw data were analyzed with the software of ABI prism 7700.

### EXAMPLE 2

### GFP expression in primary human hematopoietic cells.

Human primary T cells were positively selected from peripheral blood mononuclear fractions by panning, using mouse anti-hCD4 and anti-hCD8 monoclonal antibodies (Baxter), as previously described ¹⁸.

For primary B-cell transduction experiments, human CD19⁺ cells were positively selected from the mononuclear fraction of peripheral blood by magnetic immunoselection (Miltenyi Biotec).

Cord blood (CB) samples were obtained after informed consent. CD34⁺ cells were enriched from CB mononuclear fractions, using immunoselection with the M.A.C.S. technology, according to the manufacturer's recommendations (Miltenyi Biotec, Bergish-Gladbach, Germany); purity was usually above 95% of CD34+ cells. Sorted cells were frozen in FCS (Gibco/Invitrogen, Cergy Pontoise, France) and stored in liquid nitrogen until use.

Human primary T cells isolated from peripheral (n=2) or cord blood (n=3) samples were CD3/CD28 activated for 4 to 6 days, before parallel transduction (MOI=2) with CD19-GFP vectors and EF1-GFP control vector.

Primary T cells were activated in IMDM medium (Biowhittaker), 10% human AB serum (Institut Jacques Boy, Reims, France), 1% PS, supplemented with rhIL-2 (40ng/ml, Chiron Suresne, France) and 7µl of anti-CD3/CD28 microbeads (Dynabeads CD3/CD28 T cell expander, Dynal Biotech, Compiègne, France) per 1x10⁶ cells for 4 to 6 days before transduction.

Primary cells were transduced at a cell density of 0.3 to 0.9x10⁶ cells per well (24 well plate) in a total volume of 0.5 mL of their respective culture medium, and in the presence of protamine sulfate (4 µg/mL, Sigma Saint Quentin Fallavier, France). The lentiviral supernatant was added on the basis of 2 MOI for primary T-cells.

For primary B cells, 5 to 0.5x10⁴ CD19+ cells were cultured 5 to 7 days in 96 well plates on irradiated (75Gy) L-cells (murine fibroblasts expressing CD40-L, kindly provided by J Banchereau ²³) or LL-cells (murine fibroblasts expressing CD40-L and LIGHT; kind gift of Costello R., Marseille, France) layers in IMDM 10% human AB serum, 1% PS, supplemented with different cytokine combinations at various concentrations: rhIL-2 (20 to 50ng/mL), rhIL-4 (20 to 40ng/mL, Peproteck/Tebu) and rhIL-10 (20ng/mL, Peproteck/Tebu). Lentiviral transduction was done with the EF1-GFP vector at 50 MOI in 96 well plates coated or not with recombinant fibronectin (Chemicon/Euromedex, Mundolsheim, France), or prepared with L/LL-cells layers. GFP expression was assessed by flow cytometry 3 to 7 days after transduction.

For transduction, cord blood CD34⁺ cells were thawed, and first stimulated for 24 hours in serum-free BIT medium, 1% PS, supplemented with a combination of cytokines that includes rhFlt3-L (100 U/mL Peproteck/Tebu), rhKit-L/SCF (100 ng/mL, a kind gift of Amgen,) and rhTPO (20 ng/mL, Stem Cell Technologies). CD34⁺ progenitors were then transduced with a MOI of 50.

On day 7, 36.7 ±8% of CD3⁺ control cells expressed GFP, whereas only 4 ±0.8% and 2.8 ±1.3% of respectively S.CD19-GFP and L.CD19-GFP transduced cells showed transgene expression; GFP expression was found in a slightly higher proportion (8.9 ±1.2%) of CD3⁺ cells transduced with the M.CD19-GFP vector **(Figures 5A.5E and Table 1)**. Therefore, the promoter activity of CD 19 sequences in primary T-cells appeared low. Similar results were observed with human cord blood CD34⁺ progenitor cells. After a parallel transduction (MOI=50), 80.5 ±2.1% of EF1-GFP transduced precursors expressed GFP on day 7; in contrast, transgene expression was only observed in 3.7 to 10.9% of cells when GFP was under CD19 sequences control **(Figures 5F-5I** and **Table 1):** once again, the S and L.CD19 promoters appeared more specific when compared with M.CD19 that displayed a weak nonspecific activity.

Of interest, the Inventors were unable to efficiently express GFP transgene in human CD19+ primary B-cells. Transduction experiments were done with the EF1-GFP vector (MOI=50), using different conditions *of in vitro* activation (see material and methods for details): a maximum of 5% of GFP expressing cells was reached in any case, and did not permit a robust analysis of promoter activity in these cells. Further experiments are in progress to improve primary B-cells transduction and transgene expression efficiency, for example test of co-culture systems with murine T-helper cells (EL4.B5)³⁴.

### EXAMPLE 3

### GFP expression after in vitro differentiation of transduced CD34+ cells

Cord blood CD34⁺ transduced cells were submitted to *in vitro* differentiation in order to follow GFP expression in different hematopoietic lineages.

### 1. Clonogenic assays.

Two hundred fifty transduced CD34+ cells were plated in triplicate in 0.5 mL of methylcellulose (methocult 4230, Stem Cell Technologies) supplemented with recombinant human hematopoietic growth factors : rhEPO (2 U/mL, Eprex, Janssen-Cilag, Issy les Moulineaux, France), rhIL-3 (10 ng/mL, Peproteck/Tebu), rhIL-6 (10 ng/mL, Peproteck/Tebu), rhG-CSF (10 ng/mL, Amgen), rhGM-CSF (10ng/mL) and rhSCF (100 ng/mL). Colony-Forming Unit Granulocyte-Macrophage (CFU-GM) and Burst-Forming Unit Erythrocyte (BFU-E) were counted at day 14 according to published criteria. In some experiments, Colony-Forming Cell (CFC)-derived cells were plucked, re-suspended in phosphate buffer saline, and examined for GFP expression by flow cytometry. Single colonies were also analyzed by PCR for lentiviral transduction after pronase (Roche Applied Science) digestion (1mg/mL in 20µL Tris 50mM). Amplification was performed with two pairs of primers in order to identify the GFP transgene and the C-Kappa endogene in the same sample: GFP sens ^{5'}AAG TTC ATC TGC ACC ACC GGC (SEQ ID NO: 18) and antisens ^{5'}AAG AAG ATG GTG CGC TCC TGG (SEQ ID NO: 19) (166bp amplification); CK sens ^{5'}CCG CCA TCT GAT GAG CAG TT (SEQ ID NO: 20) and antisens ^{5'}TGA AGC TCT TTG TGA CGG GC (SEQ ID NO: 21) (273bp amplification). The PCR reaction was done at 94°C for 3' followed by 40 cycles of 45s at 94°C, 1' at 60°C, 1' at 72°C, and completed by 4' at 72°C.

An epi-fluorescence microscopic analysis of clonogenic assays revealed no expression, or at a very low frequency (<0.3%), of GFP in CFU/BFU-E, CFU-GM nor CFU-GEMM coming from progenitors transduced with S, M or L.CD19-GFP lentiviral vectors; in contrast, green colonies represented 60 ±9%, 43 ±4% and 76 ±12% of all CFU/BFU-E, CFU-GM and CFU-GEMM respectively, following transduction with the EF1-GFP control vector **(Table 2).** In order to detect possible low GFP expression in colonies that came from CD19-GFP transduced precursor cells, flow cytometry analysis of randomly picked up isolated colonies was performed: once again, no GFP expression could be observed in tested colonies (data not shown). PCR experiments carried on individual colonies showed similar level of CD34+ progenitors transduction whatever lentiviral vectors used **(Table 2),** and confirmed efficiency of the parallel transduction protocol.

### 2. Ex vivo differentiation of transduced placental progenitors

Co-cultures of human hematopoietic progenitors with the murine stromal cell line MS-5 were performed as previously described ²⁴. Briefly, MS-5 cells ²⁵ were cultured in IMDM 10% FCS, 1% PS, in 96 well plates until they reached confluence. Transduced CD34⁺ cells were then added at 3x10⁴ cells/well in IMDM supplemented with 5% human AB serum, 5% FCS (Stem Cell Technologies), 1 % PS, for B-lymphoid/myelo-monocytic differentiation. For NK cell differentiation, 1.5x10⁴ CD34⁺ cells were cultured in IMDM, 15% FCS, 1% PS supplemented with rhIL-15 (20 ng/mL, R&D system, Abingdon, UK) and rhSCF (50 ng/mL). Cells were maintained at 37°C in a fully humidified incubator, with 5% CO₂ and fed weekly by half medium change for 3 to 5 weeks. For erythroid differentiation, 2x10⁴ transduced CD34+ cells were cultured in 24 well plates in BIT medium, 1% PS, supplemented with rhIL-3 (10ng/mL), rhEPO (2U/mL) and rhSCF (100ng/mL), and maintained during 10 days at 37°C/5%CO₂. Cells were then collected for GFP and phenotypic marker expression studies with flow cytometry, using Phycoerythrin (PE) conjugated murine monoclonal antibodies (mAb) directed against human CD19, CD33, CD56 and GP-A.

Results of *in vitro* differentiation tests on MS5 cell line or in liquid culture are summarized in **Table 3,** representative assays are presented in **Figures 6A-6T** and specificity indexes are shown in Figure 7.

While CD34+ cells transduced with the EF1-GFP control vector gave an expression of GFP in all differentiated lineages - 76.2 ±6.6% of B-cells (CD19+), 62.3 ±4.9% of myeloid cells (CD33+), 36.7 ±6.1% of NK cells (CD56+) and 84 ±7.2% of erythroid cells (GPA+) - GFP expression was significant only in B-cells when differentiation came from M and L.CD19-GFP transduced progenitors: 44 ±4.9% and 36.9 ±3.7% of CD19+ cells respectively. We have to note the very low level of GFP expression when under S.CD19 sequence control, even in B-cells (4.7 ±0.8%). A relative high percentage of GFP expressing cells was found in erythroblasts (9.7 ±5.8%) when M.CD19-GFP vector was used to transduce CD34+ cell. The specificity index analysis (as described above) showed that the M and L.CD19 promoter sequences had the property to restrict GFP expression to the B-lymphoid progeny of CD34+ progenitor cells; the S.CD19 promoter appeared also relatively specific, but remained poorly efficient according to very low levels of GFP expressing B-cells. Promoter activity of CD19 sequences seemed weak even in CD19+ cells considering GFP expression intensities about 4 to 5-fold lower (MFI=30 vs. 120-150) than when the EF1 promoter was used. Of note, transduction with any of the recombinant lentiviral vectors did not appear to perturb differentiation capabilities of CD34+ progenitor cells.

### EXAMPLE 4

### GFP expression after in vivo differentiation of transduced CD34+ cells in NOD/SCID mice

Conditions were reproduced from previously published work²⁶ . Briefly, NOD/SCID mice ²⁷ were obtained from Charles River Laboratories (France). Founders were bred at the Inventors' animal facility. All animals were maintained in germ-free conditions. Seven to 8 week old animals were used for xeno-transplantation. Recipients were prepared with total body gamma irradiation at a dose of 3.25 Gy. As described by Kerre et al.²⁸, mice were injected within 24 hours of irradiation, intraperitoneally, with 0.2mg/mouse of anti CD122 monoclonal antibody (TMβ1²⁹, kindly provided by Dr T Tanaka, Tokyo, Japan). Twenty hours later, 0.05 to 0.1x10⁶ transduced or un-transduced CD34+ cells were injected via the tail vein. Ten to 12 weeks later, animals were sacrificed; bone marrow (femurs and tibia), spleen and thymus were analyzed by flow cytometry for the expression of human antigens. The presence of human cells was detected with a PE-cyanin5 (PC5) conjugated murine mAb against human CD45; cells were simultaneously stained with PE-conjugated murine mAbs that recognize different human hematopoietic differentiation antigens (CD3, CD4, CD8, CD10, CD19, CD33, CD34, IgM), and evaluated for GFP expression.

All results are given from the human To-PRO-3^{neg} and CD45+ gated cells. The expression of marker was up to 76% in any types of analyzed cells in bone marrow or spleen of mice engrafted by EF1-GFP transduced progenitor cells with a strong expression of GFP (MFI∼ 1-2x10³) **(Figures 8A-8ZA, Table 4).** In bone marrow, with S.CD19-GFP transduced cells, the expression of marker gene appeared similar in all differentiated human lineages (about 20% of GFP expressing cells) **(Table 4).** Detailed analysis of S.CD19 sequence behavior *in vivo* showed that global GFP expression (varying from1 to 81.7% in hCD45+ cells) as specificity of GFP expression (0<SI<6.07 for CD33+ cells) was strongly variable between animals. In contrast, transgene expression was found preferentially in B-lymphoid progeny of M or L.CD9-GFP transduced progenitor cells. In bone marrow, 65.0 ±7.6% and 70.2 ±5.0% of CD19+ cells expressed GFP to only 30.8 ±4.9% and 15.9 ±4.1% of CD33+ cells for M and L.CD19-GFP transduced cells respectively **(Figures 8A-8ZA, Table 4);** to notice, CD10+ B-cell compartment followed CD19+ cells GFP expression profile. In spleen, a specific GFP expression was observed in CD19+ and more mature IgM+ B-cells compared with CD33+ myeloid cells when the M and L.CD19 sequences were used **(Figures 8A-8ZA, Table 4)**. All CD19 promoters gave similar GFP expression intensities in bone marrow or spleen B-cell progeny (MFI ~ 150). Marker gene expression under control of the different used promoters in human differentiated cells was summarize in the specificity index histograms: once more, the L.CD19 promoter sequence showed better specificity for B-cell compartment than the M.CD19 one (0.23 ±0.05% vs. 0.49 ±0.05% for bone marrow CD33+ SI; SI values were similar in spleen) **(Figures 9A, 9B)**; interestingly, less variations of GFP positive B-cells percentages were observed between animals when L.CD19 sequence was used that suggested a better promoter functionality for this sequence. First analysis of global bone marrow CD34+ population showed GFP expression in a large part of these cells even when they came from M or L.CD19-GFP transduced progenitors **(Figures 8A-8ZA, Table 4).** However, a more precise study of this progenitor pool revealed that transgene expression was in majority found in CD34+/CD19+ immature B-cells and only weakly in CD34+/CD19-non-B progenitor-cells **(Figure 10, *Table 5*):** this was an obvious result for L.CD19-GFP transduced cells (62 ±8.8% vs. 17.2 ±5.4% GFP expressing cells respectively) whereas specificity appeared weaker in M.CD19-GFP transduced cells (52.5 ±12.4% vs. 48.6 ±9.7% GFP expressing cells respectively).

Of note, thymus engraftment was a rare event (10/34 bone marrow engrafted tested mice) and weakly pronounced (hCD45+ cells<1% of total). Moreover, when human cells were identified, there was not systematically presence of CD3+ T-lymphoid cells. In the case reported **Figure 11A-11D.** where appeared CD3, CD4 and CD8+ cells in a mouse engrafted with M.CD19-GFP transduced progenitor cells, only background expression of marker was observed on former T-lymphoid cells and GFP expressing cells were mainly found in CD19+ population.

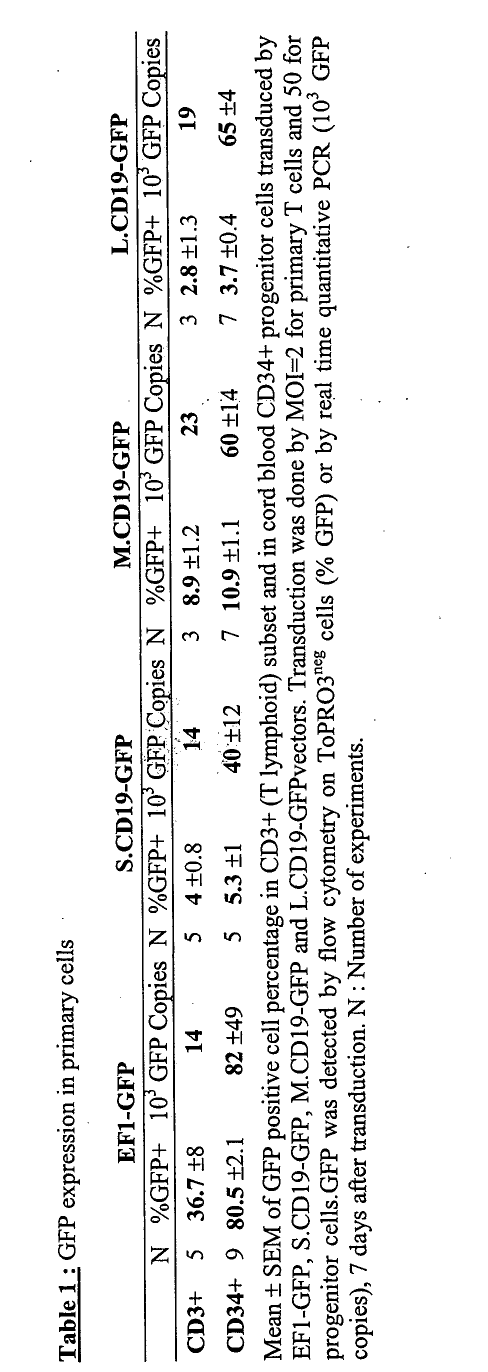

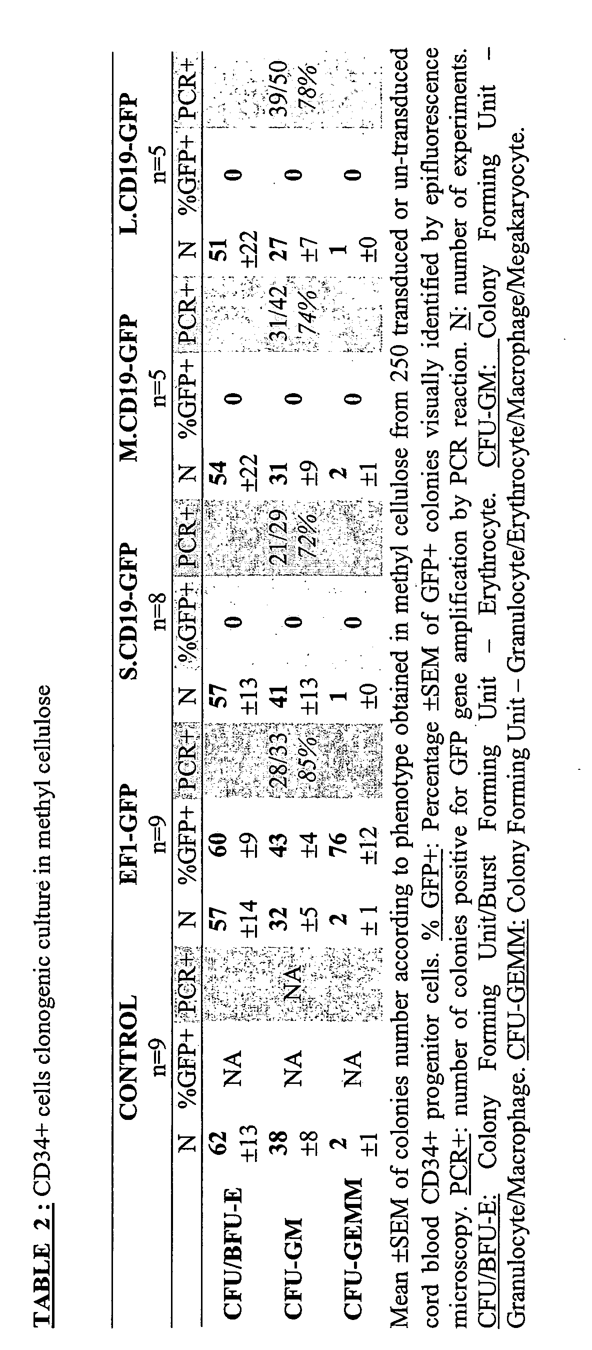

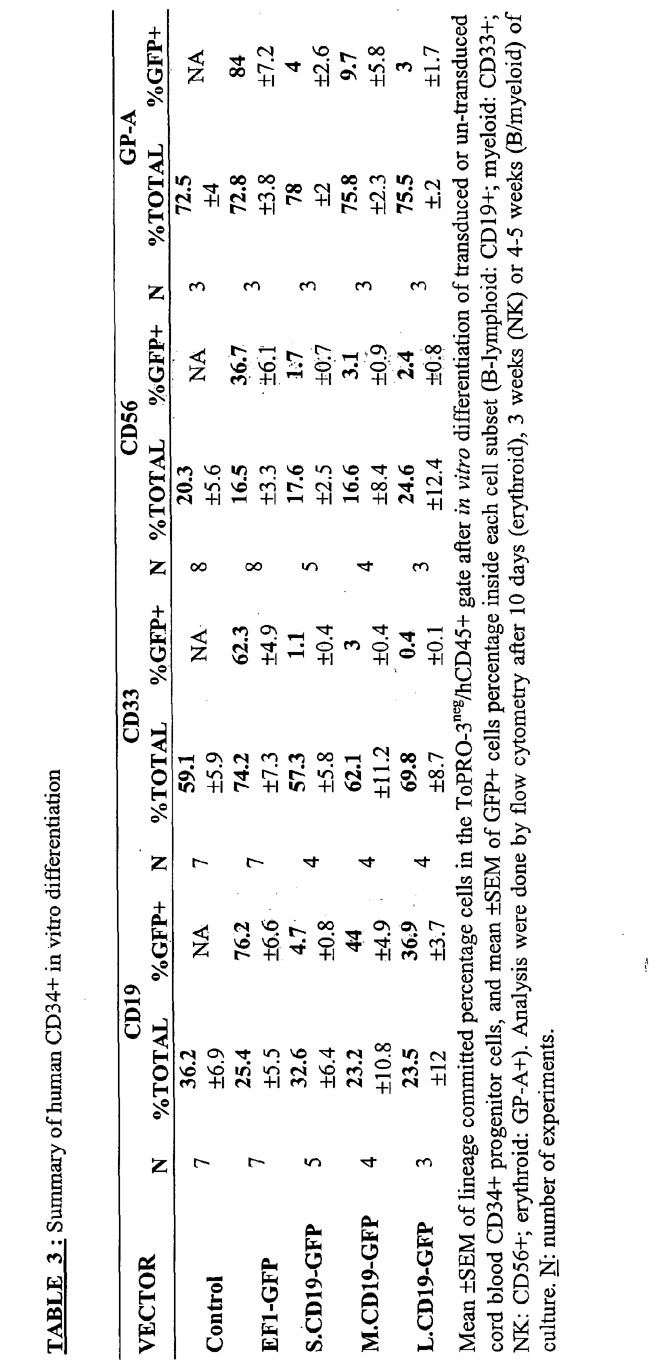

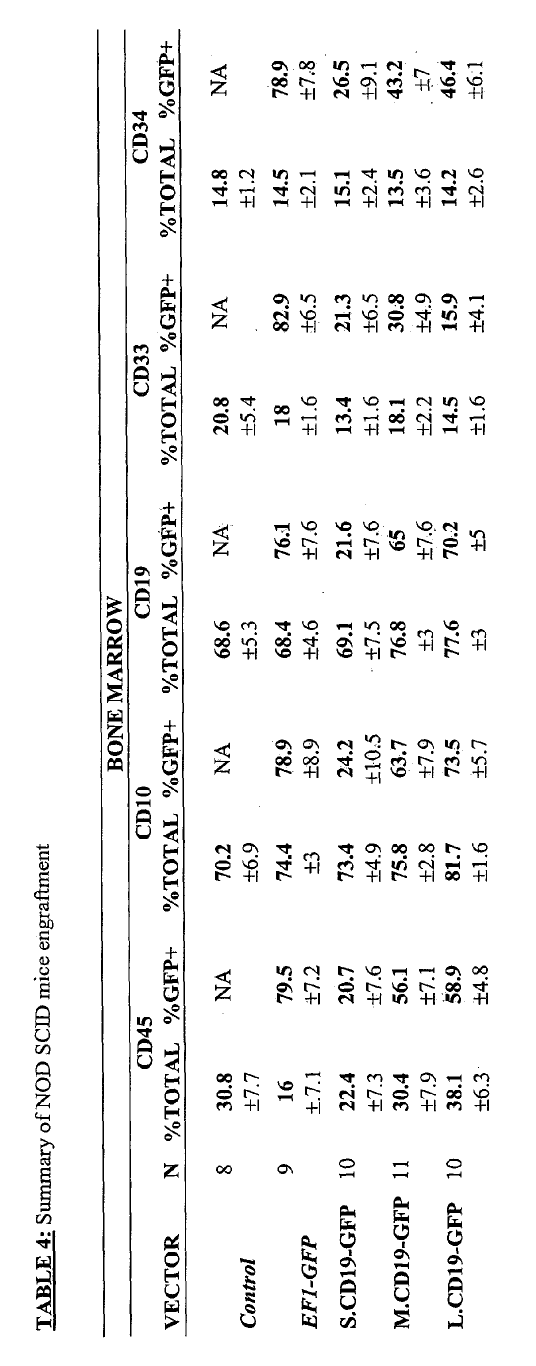

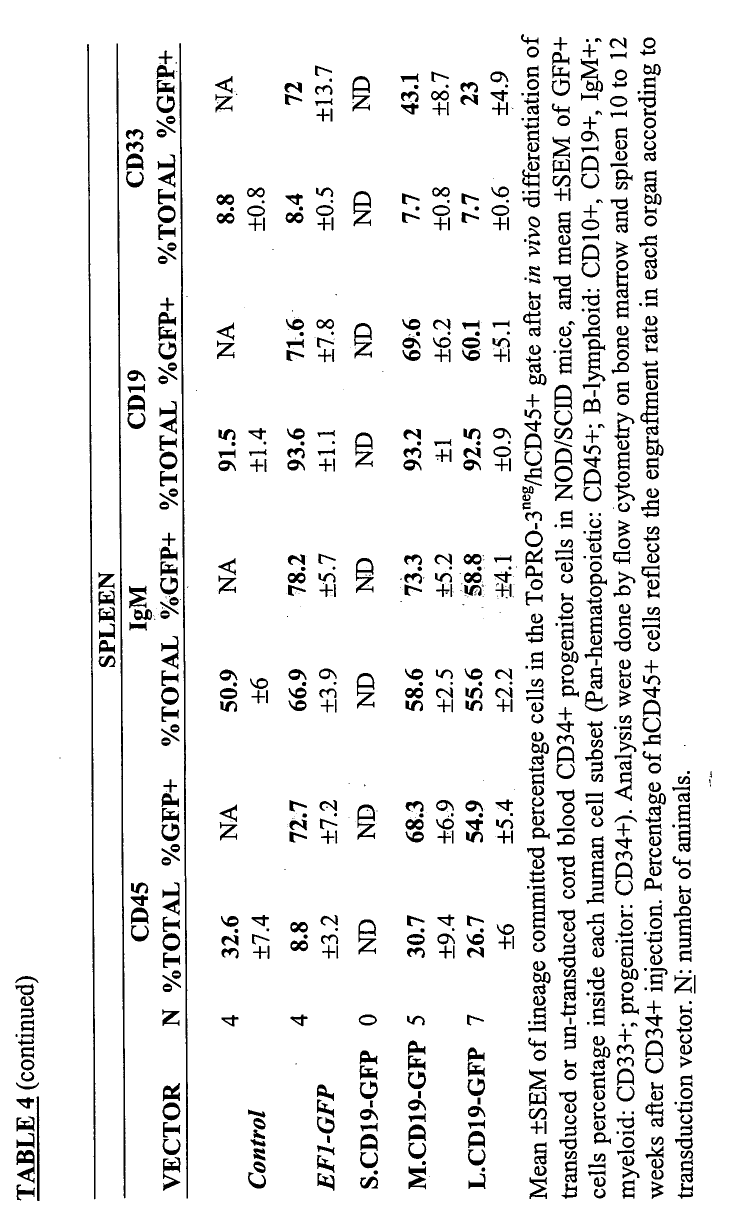

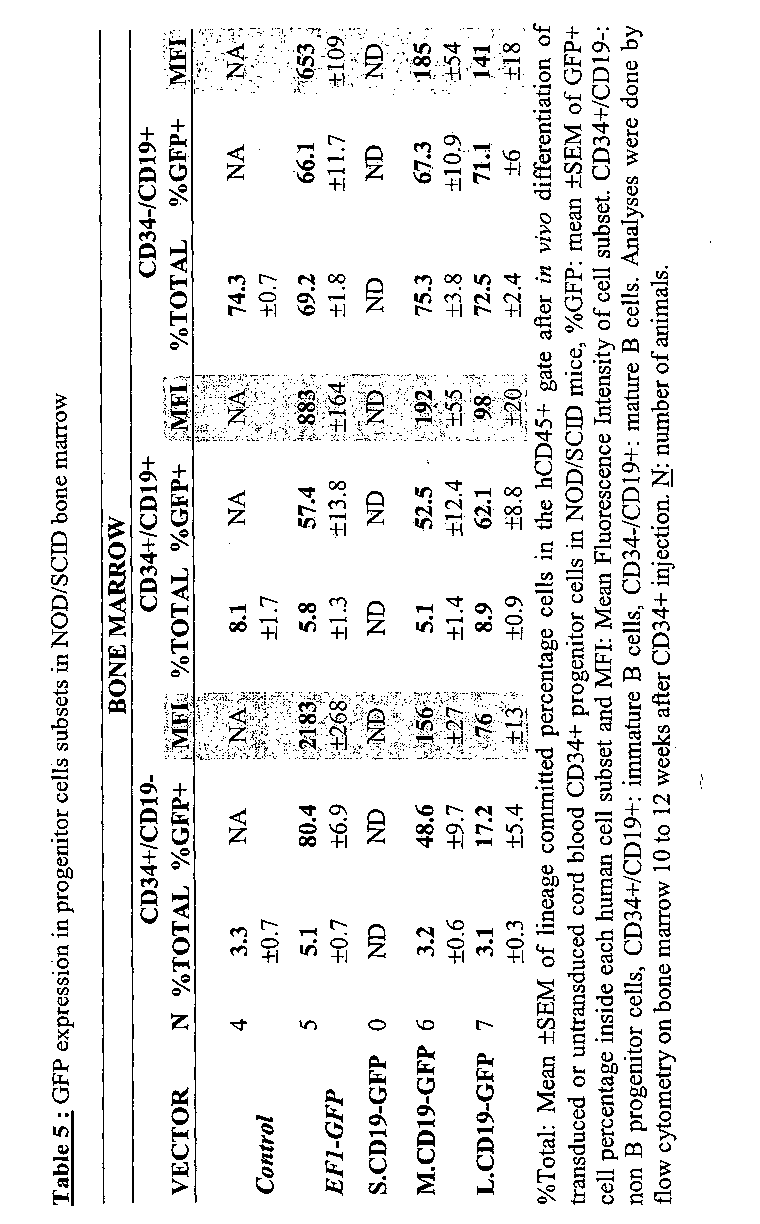

### REFERENCES

1. Naldini L, Blomer U, Gallay P, Ory D, Mulligan R, Gage FH, Verma IM, Trono D: In vivo gene delivery and stable transduction of nondividing cells by a lentiviral vector. Science 272:263, 1996
2. Vigna E, Naldini L: Lentiviral vectors: excellent tools for experimental gene transfer and promising candidates for gene therapy. J Gene Med 2:308, 2000
3. Sirven A, Ravet E, Charneau P, Zennou V, Coulombel L, Guetard D, Pflumio F, Dubart-Kupperschmitt A: Enhanced transgene expression in cord blood cd34(+)-derived hematopoietic cells, including developing t cells and nod/scid mouse repopulating cells, following transduction with modified trip lentiviral vectors. Mol Ther 3:438, 2001
4. Guenechea G, Gan OI, Inamitsu T, Dorrell C, Pereira DS, Kelly M, Naldini L, Dick JE: Transduction of human CD34+ CD38- bone marrow and cord blood-derived SCID-repopulating cells with third-generation lentiviral vectors. Mol Ther 1:566, 2000
5. Indraccolo S, Minuzzo S, Roccaforte F, Zamarchi R, Habeler W, Stievano L, Tosello V, Klein D, Gunzburg WH, Basso G, Chieco-Bianchi L, Amadori A: Effects of CD2 locus control region sequences on gene expression by retroviral and lentiviral vectors. Blood 98:3607, 2001
6. Marodon G, Mouly E, Blair EJ, Frisen C, Lemoine FM, Klatzmann D: Specific transgene expression in human and mouse CD4+ cells using lentiviral vectors with regulatory sequences from the CD4 gene. Blood 101:3416, 2003
7. Cui Y, Golob J, Kelleher E, Ye Z, Pardoll D, Cheng L: Targeting transgene expression to antigen-presenting cells derived from lentivirus-transduced engrafting human hematopoietic stem/progenitor cells. Blood 99:399, 2002
8. Gough PJ, Raines EW: Gene therapy of apolipoprotein E-deficient mice using a novel macrophage-specific retroviral vector. Blood 101:485, 2003
9. Moreau-Gaudry F, Xia P, Jiang G, Perelman NP, Bauer G, Ellis J, Surinya KH, Mavilio F, Shen CK, Malik P : High-level erythroid-specific gene expression in primary human and murine hematopoietic cells with self-inactivating lentiviral vectors. Blood 98 : 2664, 2001
10. Imren S, Payen E, Westerman KA, Pawliuk R, Fabry ME, Eaves CJ, Cavilla B, Wadsworth LD, Beuzard Y, Bouhassira EE, Russell R, London IM, Nagel RL, Leboulch P, Humphries RK: Permanent and panerythroid correction of murine beta thalassemia by multiple lentiviral integration in hematopoietic stem cells. PNAS 99 : 14380,2002
11. Kozmik Z, Wang S, Dorfler P, Adams B, Busslinger M: The promoter of the CD19 gene is a target for the B-cell-specific transcription factor BSAP. Mol.Cell Biol. 12:2662, 1992
12. Tedder TF, Inaoki M, Sato S: The CD19-CD21 complex regulates signal transduction thresholds governing humoral immunity and autoimmunity. Immunity 6:107, 1997
13. Zhou LJ, Smith HM, Waldschmidt TJ, Schwarting R, Daley J, Tedder TF: Tissue-specific expression of the human CD 19 gene in transgenic mice inhibits antigen-independent B-lymphocyte development. Mol Cell Biol 14:3884, 1994
14. Maas A, Dingjan GM, Grosveld F, Hendriks RW: Early arrest in B cell development in transgenic mice that express the E41K Bruton's tyrosine kinase mutant under the control of the CD19 promoter region. J.Immunol. 162:6526, 1999
15. Kleindienst P, Chretien I, Winkler T, Brocker T: Functional comparison of thymic B cells and dendritic cells in vivo. Blood 95:2610, 2000
16. Riva A, Wilson GL, Kehrl JH: In vivo footprinting and mutational analysis of the proximal CD19 promoter reveal important roles for an SP1/Egr-1 binding site and a novel site termed the PyG box. J.Immunol. 159:1284, 1997
17. Lutzko C, Senadheera D, Skelton D, Petersen D, Kohn DB: Lentivirus vectors incorporating the immunoglobulin heavy chain enhancer and matrix attachment regions provide position-independent expression in B lymphocytes. J Virol 77:7341, 2003
18. Defrance T, Vanbervliet B, Durand I, Briolay J, Banchereau J: Proliferation and differentiation of human CD5⁺ and CD5⁻ B cell subsets activated through their antigen receptors or CD40 antigens. Eur.J.Immunol. 22:2831, 1992
19. Hubbard T, Barker D, Birney E, Cameron G, Chen Y, Clark L, Cox T, Cuff J, Curwen V, Down T, Durbin R, Eyras E, Gilbert J, Hammond M, Huminiecki L, Kasprzyk A, Lehvaslaiho H, Lijnzaad P, Melsopp C, Mongin E, Pettett R, Pocock M, Potter S, Rust A, Schmidt E, Searle S, Slater G, Smith J, Spooner W, Stabenau A, Stalker J, Stupka E, Ureta-Vidal A, Vastrik I, Clamp M: The Ensembl genome database project. Nucleic Acids Res 30:38, 2002
20. Quandt K, Frech K, Karas H, Wingender E, Werner T: MatInd and MatInspector: new fast and versatile tools for detection of consensus matches in nucleotide sequence data. Nucleic Acids Res 23:4878, 1995
21. Schwartz S, Zhang Z, Frazer KA, Smit A, Riemer C, Bouck J, Gibbs R, Hardison R, Miller W: PipMaker--a web server for aligning two genomic DNA sequences. Genome Res 10:577, 2000
22. Rocancourt D, Bonnerot C, Jouin H, Emerman M, Nicolas JF: Activation of a beta-galactosidase recombinant provirus: application to titration of human immunodeficiency virus (HIV) and HIV-infected cells. J Virol 64:2660, 1990
23. Garrone P, Neidhardt EM, Garcia E, Galibert L, Van Kooten C, Banchereau J: Fas ligation induces apoptosis of CD40-activated human B lymphocytes. J.Exp.Med. 182:1265, 1995
24. Hacein-Bey S, Gross F, Nusbaum P, Hue C, Hamel Y, Fischer A, Cavazzana-Calvo M: Optimization of retroviral gene transfer protocol to maintain the lymphoid potential of progenitor cells. Hum Gene Ther 12:291, 2001
25. Itoh K, Tezuka H, Sakoda H, Konno M, Nagata K, Uchiyama T, Uchino H, Mori KJ: Reproducible establishment of hemopoietic supportive stromal cell lines from murine bone marrow. Exp Hematol 17:145, 1989
26. Tonnelle C, Bardin F, Maroc C, Imbert AM, Campa F, Dalloul A, Schmitt C, Chabannon C: Forced expression of the Ikaros 6 isoform in human placental blood CD34(+) cells impairs their ability to differentiate toward the B-lymphoid lineage. Blood 98:2673, 2001
27. Shultz LD, Schweitzer PA, Christianson SW, Gott B, Schweitzer IB, Tennent B, McKenna S, Mobraaten L, Rajan TV, Greiner DL: Multiple defects in innate and adaptive immunologic function in NOD/LtSz-scid mice. J.Immunol. 154:180, 1995
28. Kerre TC, De Smet G, De Smedt M, Zippelius A, Pittet MJ, Langerak AW, De Bosscher J, Offner F, Vandekerckhove B, Plum J: Adapted NOD/SCID model supports development of phenotypically and functionally mature T cells from human umbilical cord blood CD34(+) cells. Blood 99:1620, 2002
29. Tanaka T, Kitamura F, Nagasaka Y, Kuida K, Suwa H, Miyasaka M: Selective long-term elimination of natural killer cells in vivo by an anti-interleukin 2 receptor beta chain monoclonal antibody in mice. J Exp Med 178:1103, 1993
30. Pennacchio LA, Rubin EM: Genomic strategies to identify mammalian regulatory sequences. Nat Rev Genet 2:100, 2001
31. Kozmik Z, Wang S, Dorfler P, Adams B, Busslinger M: The promoter of the CD19 gene is a target for the B-cell-specific transcription factor BSAP. Mol.Cell.Biol. 12:2662, 1992
32. Gisler R, Akerblad P, Sigvardsson M: A human early B-cell factor-like protein participates in the regulation of the human CD19 promoter. Mol Immunol 36:1067, 1999
33. Kehrl JH, Riva A, Wilson GL, Thévenin C: Molecular mechanisms regulating CD19, CD20 and CD22 gene expression. Immunol.Today 15:432, 1994
34. Bovia F, Salmon P, Matthes T, Kvell K, Nguyen TH, Werner-Favre C, Barnet M, Nagy M, Leuba F, Arrighi JF, Piguet V, Trono D, Zubler RH: Efficient transduction of primary human B lymphocytes and nondividing myeloma B cells with HIV-1-derived lentiviral vectors. Blood 101:1727,2003

## Claims

1. A nucleic acid sequence comprising or consisting of:
• a S sequence, said S sequence comprising or consisting of:
(1) a first sequence corresponding to a fragment of at least 10 nucleotides of the promoter sequence of the human CD19 gene, such as defined in Figure 1, the ends of which being comprised from the nucleotide at position -1500 to the nucleotide at position -1000 of the promoter sequence of the human CD19 gene,
(2) a second sequence consisting of the contiguous nucleotides ranging from position -231 to position -1 (SEQ ID NO :1) of the promoter sequence of the human CD 19 gene; or
• a sequence derived from the above defined S sequence by insertion, deletion or mutation of at least one nucleotide, or presenting an homology of at least 60 % per block of 150 to 200 contiguous nucleotides with the above defined S sequence, provided that said sequence is capable of directing the specific expression of a gene in cells of the B lymphoid lineage;
provided that when said S sequence consists of the contiguous nucleotides of the promoter sequence of the human CD19 gene ranging from the 5' end of the first sequence to position -1, then, if present, the 5' contiguous nucleotides flanking the 5'end of the first sequence in said nucleic acid sequence are different from the 5' contiguous nucleotides flanking the 5' end of the sequence corresponding to said first sequence in the promoter sequence of the human CD 19 gene.

2. A nucleic acid sequence according to claim 1, **characterised in that** the S sequence consists of:
(1) a first sequence consisting of the contiguous nucleotides ranging from position X to position Y of the promoter sequence of the human CD19 gene, X and Y being negative integer numbers, and
(2) a second sequence consisting of contiguous nucleotides ranging from position Z to position -1 of the promoter sequence of the human CD19 gene, Z being a negative integer number,
wherein X, Y and Z are such, that:
X ranges from -1500 to -1000,
Y ranges from X+9 to Z-1, and
Z ranges from Y+1 to -231,
the 3' end of said first sequence being covalently linked to the 5' end of said second sequence.

3. A nucleic acid sequence according to claim 2, **characterized in that**
X = -1274
Y ranges from -1042 to Z-1, and
Z ranges from Y+1 to -231.

4. A nucleic acid sequence according to claim 3, **characterized in that**
X = -1274,
Y = -1042, and
Z = -231,
that is, a nucleic acid sequence comprising or consisting of a S sequence, said S sequence consisting of:
(1) a first sequence consisting of the contiguous nucleotides ranging from position -1274 to position -1042 (SEQ ID NO: 2) of the promoter sequence of the human CD19 gene, and
(2) a second sequence consisting of the contiguous nucleotide ranging from position -231 to position -1 (SEQ ID NO: 1) of the promoter sequence of the human CD19 gene,
the 3' end of said first sequence being covalently linked to the 5' end of said second sequence, said S sequence corresponding to SEQ ID NO: 3.

5. A nucleic acid sequence according to claim 2 or 3, **characterized in that**
X is equal to -1274, and
Y = Z-1,
that is, a nucleic acid sequence comprising or consisting of a S sequence, said S sequence consisting of the contiguous nucleotides ranging from position -1274 to position -1 (SEQ ID NO: 4) of the promoter sequence of the human CD19 gene, or of a sequence (SEQ ID NO: 5) derived from said S sequence by the deletion of one nucleotide selected from the contiguous nucleotides ranging from position -541 to position -530 of the promoter sequence of the human CD19 gene, provided that, if present, the 5' contiguous nucleotides flanking the 5'end of the S sequence in said nucleic acid sequence are different from the 5' contiguous nucleotides flanking the nucleotide at position -1274 in the promoter sequence of the human CD 19 gene.

6. An expression sequence, **characterized in that** it comprises a nucleic acid sequence according to any of claims 1 to 5, linked to a protein encoding nucleic acid sequence, in particular a protein encoding nucleic acid sequence coding for a protein expressed specifically in cells of the B lymphoid lineage, such as a sequence encoding Btk, Igα, surrogate light chains, BLNK, Igb, or to a hairpin RNA encoding nucleic acid sequence, or to a suicide gene such as the HSVTK gene (Herpes simplex virus thymidine kinase), or to a marker gene such as the GFP gene (Green fluorescent protein) or the LacZ gene.

7. An expression sequence according to claim 6, **characterized in that** the nucleic acid sequence consists of SEQ ID NO: 3, 4 or 5.

8. A vector, in particular an expression vector, **characterized in that** it comprises a nucleic acid sequence or an expression sequence according to any of claims 1 to 7.

9. A vector, in particular an expression vector, according to claim 8, **characterized in that** the nucleic acid sequence consists of SEQ ID NO: 3, 4 or 5, such as the lentiviral vectors corresponding to SEQ ID NO: 6, 7 and 8.

10. A cell, in particular a cell of the B lymphoid lineage, comprising a nucleic acid sequence according to any of claims 1 to 5, or an expression sequence according to claim 6 or 7, or a vector according to claim 8 or 9.

11. A cell, in particular a cell of the B lymphoid lineage, according to claim 10, **characterized in that** it comprises a nucleic acid sequence consisting of SEQ ID NO: 3, 4 or 5.

12. A nonhuman transgenic animal, **characterized in that** all or some of its cells comprise a nucleic acid sequence according to any of claims 1 to 5, or an expression sequence according to claim 6 or 7, or a vector according to claim 8 or 9, said animal being in particular a mammal, such as a mouse or a rat.

13. A pharmaceutical composition comprising as active substance a nucleic acid sequence according to any of claims 1 to 5, or an expression sequence according to claim 6 or 7, or a vector according to claim 8 or 9, or a cell according to claim 10 or 11, in association with a pharmaceutically acceptable vehicle.

14. The use of a nucleic acid sequence according to any of claims 1 to 5, or an expression sequence according to claim 6 or 7, or a vector according to claim 8 or 9, or a cell according to claim 10 or 11, for the manufacture of a medicament intended to treat pathologies in which cells of the B lymphoid lineage are involved, such as autoimmune diseases, B lymphomas, B leukemias, antibody deficiencies, in particular X linked agammaglobulinemia (Bruton's disease), or diseases resulting from a deficient preB cell receptor complex, such as a Igα defect, a surrogate light chains defect, or a BLNK defect.


SEQUENCE LISTING

<110> INSTITUT NATIONAL DE LA SANTE ET DE LA RECHERCHE MEDICALE

<120> B LYMPHOID LINEAGE SPECIFIC PROMOTER SEQUENCES AND PHARMACEUTICAL
COMPOSITIONS CONTAINING THE SAME

<130> IFB 03 CK INS PROM

<160> 21

<170> PatentIn version 3.1

<210> 1
<211> 231
<212> DNA
<213> Homo sapiens

<400> 1
tcccgtggta gtgagagctg ggatgaaaat aaaacagcgt ggcagggagg aggcaagtgt 60

tgtgagtctg gagggttcct ggagaatggg gcctgaggcg tgaccaccgc cttcctctct 120

ggggggactg cctgccgccc ccgcagacac ccatggttga gtgccctcca ggcccctgcc 180

tgccccagca tcccctgcgc gaagctgggt gccccggaga gtctgaccac c 231

<210> 2
<211> 233
<212> DNA
<213> Homo sapiens

<400> 2
aatgagggga ttagagagat tttgttgaat gaaaggcaga ttgagtcctg ctactcgccc 60

ccttcattcc ccttcattca tgcctcattc ttccgcctcc cagccgcctc aactggccaa 120

agggaagtgg aggccctgcc acctgtaggg agggtcccct ggggcttgcc cacagcaaac 180

aggaagtcac agcctggtga gatgggcctg ggaatcagcc actgagaaag tgg 233

<210> 3
<211> 464
<212> DNA
<213> Artificial sequence

<220>
<223> Composite sequence derived from the CD19 promoter

<400> 3
aatgagggga ttagagagat tttgttgaat gaaaggcaga ttgagtcctg ctactcgccc 60

ccttcattcc ccttcattca tgcctcattc ttccgcctcc cagccgcctc aactggccaa 120

agggaagtgg aggccctgcc acctgtaggg agggtcccct ggggcttgcc cacagcaaac 180

aggaagtcac agcctggtga gatgggcctg ggaatcagcc actgagaaag tggtcccgtg 240

gtagtgagag ctgggatgaa aataaaacag cgtggcaggg aggaggcaag tgttgtgagt 300

ctggagggtt cctggagaat ggggcctgag gcgtgaccac cgccttcctc tctgggggga 360

ctgcctgccg cccccgcaga cacccatggt tgagtgccct ccaggcccct gcctgcccca 420

gcatcccctg cgcgaagctg ggtgccccgg agagtctgac cacc 464

<210> 4
<211> 1274
<212> DNA
<213> Homo sapiens

<400> 4
aatgagggga ttagagagat tttgttgaat gaaaggcaga ttgagtcctg ctactcgccc 60

ccttcattcc ccttcattca tgcctcattc ttccgcctcc cagccgcctc aactggccaa 120

agggaagtgg aggccctgcc acctgtaggg agggtcccct ggggcttgcc cacagcaaac 180

aggaagtcac agcctggtga gatgggcctg ggaatcagcc actgagaaag tgggtctctt 240

gggtccctga attctttttc tgagtccctg cagcagtgaa aaagacacag aggcacatag 300

agagtgacag agaaagagag agacagagag gagaggcatg gggcagaata agaacagatt 360

taggagttag aactcctggg ttcttttaaa acaatttttc ttttagagac agggtcttgt 420

tgtgttgccc ggactggagc acagtggcta ttcccaggca taatcatggt gcactgcagc 480

cttgaactcc tgggctcaag cgatccttct acctcagcct cccaaggacc tgggaccata 540

ggcgtgtacc actgtgcctg gcttttgcct ggttttaaac tgaggcagta tgacttgagc 600

tcttaggcat taattgaagc tgtatctcat taactgaggg cttatgatgt gctggacact 660

gggctaatag tgctgaacat attgtcattt ttaatcttca caaacaatat ttgtatagga 720

ctgttttctt ttcttttttt tttttgaaac agagtctcac tctggtgccc aggctggagt 780

gcagtggtgt gatctcggct cactgcaacc tccgcctcct ggtttccagt gattctcctg 840

cctcagcctc ctaagtagct gggattacag gtgtgcgcca ccatgcccgg ctaatttttt 900

tttttttttt tgagaaggag tctatgtgcc cagcattgtt ctagagcact tgcaattagt 960

ggtgaacaac acggtctcta ctccaagggg ctcacattct tgtgcagaaa acagaaatga 1020

acaaataaac acacaagatc atttcccgtg gtagtgagag ctgggatgaa aataaaacag 1080

cgtggcaggg aggaggcaag tgttgtgagt ctggagggtt cctggagaat ggggcctgag 1140

gcgtgaccac cgccttcctc tctgggggga ctgcctgccg cccccgcaga cacccatggt 1200

tgagtgccct ccaggcccct gcctgcccca gcatcccctg cgcgaagctg ggtgccccgg 1260

agagtctgac cacc 1274

<210> 5
<211> 1273
<212> DNA
<213> Artificial sequence

<220>
<223> Deleted sequence deriving from SEQ ID NO 4

<400> 5
   aatgagggga ttagagagat tttgttgaat gaaaggcaga ttgagtcctg ctactcgccc 60
   ccttcattcc ccttcattca tgcctcattc ttccgcctcc cagccgcctc aactggccaa 120
   agggaagtgg aggccctgcc acctgtaggg agggtcccct ggggcttgcc cacagcaaac 180
   aggaagtcac agcctggtga gatgggcctg ggaatcagcc actgagaaag tgggtctctt 240
   gggtccctga attctttttc tgagtccctg cagcagtgaa aaagacacag aggcacatag 300
   agagtgacag agaaagagag agacagagag gagaggcatg gggcagaata agaacagatt 360
   taggagttag aactcctggg ttcttttaaa acaatttttc ttttagagac agggtcttgt 420
   tgtgttgccc ggactggagc acagtggcta ttcccaggca taatcatggt gcactgcagc 480
   cttgaactcc tgggctcaag cgatccttct acctcagcct cccaaggacc tgggaccata 540
   ggcgtgtacc actgtgcctg gcttttgcct ggttttaaac tgaggcagta tgacttgagc 600
   tcttaggcat taattgaagc tgtatctcat taactgaggg cttatgatgt gctggacact 660
   gggctaatag tgctgaacat attgtcattt ttaatcttca caaacaatat ttgtatagga 720
   ctgttttctt ttcttttttt ttttgaaaca gagtctcact ctggtgccca ggctggagtg 780
   cagtggtgtg atctcggctc actgcaacct ccgcctcctg gtttccagtg attctcctgc 840
   ctcagcctcc taagtagctg ggattacagg tgtgcgccac catgcccggc taattttttt 900
   tttttttttt gagaaggagt ctatgtgccc agcattgttc tagagcactt gcaattagtg 960
   gtgaacaaca cggtctctac tccaaggggc tcacattctt gtgcagaaaa cagaaatgaa 1020
   caaataaaca cacaagatca tttcccgtgg tagtgagagc tgggatgaaa ataaaacagc 1080
   gtggcaggga ggaggcaagt gttgtgagtc tggagggttc ctggagaatg gggcctgagg 1140
   cgtgaccacc gccttcctct ctggggggac tgcctgccgc ccccgcagac acccatggtt 1200
   gagtgccctc caggcccctg cctgccccag catcccctgc gcgaagctgg gtgccccgga 1260
   gagtctgacc acc 1273

<210> 6
<211> 3037
<212> DNA
<213> Artificial sequence

<220>
<223> Lentiviral vector containing a promoter derived from the human CD
      19 promoter

<400> 6
tggaagggct aattcactcc caaagaagac aagatatcct tgatctgtgg atctaccaca 60

cacaaggcta cttccctgat tagcagaact acacaccagg gccaggggtc agatatccac 120

tgacctttgg atggtgctac aagctagtac cagttgagcc agataaggta gaagaggcca 180

ataaaggaga gaacaccagc ttgttacacc ctgtgagcct gcatgggatg gatgacccgg 240

agagagaagt gttagagtgg aggtttgaca gccgcctagc atttcatcac gtggcccgag 300

agctgcatcc ggagtacttc aagaactgct gatatcgagc ttgctacaag ggactttccg 360

ctggggactt tccagggagg cgtggcctgg gcgggactgg ggagtggcga gccctcagat 420

cctgcatata agcagctgct ttttgcctgt actgggtctc tctggttaga ccagatctga 480

gcctgggagc tctctggcta actagggaac ccactgctta agcctcaata aagcttgcct 540

tgagtgcttc aagtagtgtg tgcccgtctg ttgtgtgact ctggtaacta gagatccctc 600

agaccctttt agtcagtgtg gaaaatctct agcagtggcg cccgaacagg gacttgaaag 660

cgaaagggaa accagaggag ctctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcagta ttaagcgggg gagaattaga tcgcgatggg 840

aaaaaattcg gttaaggcca gggggaaaga aaaaatataa attaaaacat atagtatggg 900

caagcaggga gctagaacga ttcgcagtta atcctggcct gttagaaaca tcagaaggct 960

gtagacaaat actgggacag ctacaaccat cccttcagac aggatcagaa gaacttagat 1020

cattatataa tacagtagca accctctatt gtgtgcatca aaggatagag ataaaagaca 1080

ccaaggaagc tttagacaag atagaggaag agcaaaacaa aagtaagacc accgcacagc 1140

aagcggccgc tgatcttcag acctggagga ggagatatga gggacaattg gagaagtgaa 1200

ttatataaat ataaagtagt aaaaattgaa ccattaggag tagcacccac caaggcaaag 1260

agaagagtgg tgcagagaga aaaaagagca gtgggaatag gagctttgtt ccttgggttc 1320

ttgggagcag caggaagcac tatgggcgca gcgtcaatga cgctgacggt acaggccaga 1380

caattattgt ctggtatagt gcagcagcag aacaatttgc tgagggctat tgaggcgcaa 1440

cagcatctgt tgcaactcac agtctggggc atcaagcagc tccaggcaag aatcctggct 1500

gtggaaagat acctaaagga tcaacagctc ctggggattt ggggttgctc tggaaaactc 1560

atttgcacca ctgctgtgcc ttggaatgct agttggagta ataaatctct ggaacagatt 1620

tggaatcaca cgacctggat ggagtgggac agagaaatta acaattacac aagcttaata 1680

cactccttaa ttgaagaatc gcaaaaccag caagaaaaga atgaacaaga attattggaa 1740

ttagataaat gggcaagttt gtggaattgg tttaacataa caaattggct gtggtatata 1800

aaattattca taatgatagt aggaggcttg gtaggtttaa gaatagtttt tgctgtactt 1860

tctatagtga atagagttag gcagggatat tcaccattat cgtttcagac ccacctccca 1920

accccgaggg gacccgacag gcccgaagga atagaagaag aaggtggaga gagagacaga 1980

gacagatcca ttcgattagt gaacggatct cgacggtatc gccgaattca caaatggcag 2040

tattcattca caattttaaa agaaaagggg ggattggggg gtacagtgca ggggaaagaa 2100

taatagacat aatagcaaca gatatacaaa ctacagaact acaaaaacaa attataaaaa 2160

ttcaaaattt tcgggtttat tacagagaca gcagagaccc tatttggctg atacgcgtaa 2220

tgaggggatt agagagattt tgttgaatga aaggcagatt gagtcctgct actcgccccc 2280

ttcattcccc ttcattcatg cctcattctt ccgcctccca gccgcctcaa ctggccaaag 2340

ggaagtggag gccctgccac ctgtagggag ggtcccctgg ggcttgccca cagcaaacag 2400

gaagtcacag cctggtgaga tgggcctggg aatcagccac tgagaaagtg gtcccgtggt 2460

agtgagagct gggatgaaaa taaaacagcg tggcagggag gaggcaagtg ttgtgagtct 2520

ggagggttcc tggagaatgg ggcctgaggc gtgaccaccg ccttcctctc tggggggact 2580

gcctgccgcc cccgcagaca cccatggttg agtgccctcc aggcccctgc ctgccccagc 2640

atcccctgcg cgaagctggg tgccccggag agtctgacca ccaggatccc tcgagaggga 2700

ttcgagctcg gtacctttaa gaccaatgac ttacaaggca gctgtagatc ttagccactt 2760

tttaaaagaa aaggggggac tggaagggct aattcactcc caacgaagac aagattcgag 2820

agatgctgca tataagcagc tgctttttgc ttgtactggg tctctctggt tagaccagat 2880

ctgagcctgg gagctctctg gctaactagg gaacccactg cttaagcctc aataaagctt 2940

gccttgagtg cttcaagtag tgtgtgcccg tctgttgtgt gactctggta actagagatc 3000

cctcagaccc ttttagtcag tgtggaaaat ctctagc 3037

<210> 7
<211> 3847
<212> DNA
<213> Artificial sequence

<220>
<223> Lentiviral vector containing a promoter derived from the human CD
      19 promoter

<400> 7
tggaagggct aattcactcc caaagaagac aagatatcct tgatctgtgg atctaccaca 60

cacaaggcta cttccctgat tagcagaact acacaccagg gccaggggtc agatatccac 120

tgacctttgg atggtgctac aagctagtac cagttgagcc agataaggta gaagaggcca 180

ataaaggaga gaacaccagc ttgttacacc ctgtgagcct gcatgggatg gatgacccgg 240

agagagaagt gttagagtgg aggtttgaca gccgcctagc atttcatcac gtggcccgag 300

agctgcatcc ggagtacttc aagaactgct gatatcgagc ttgctacaag ggactttccg 360

ctggggactt tccagggagg cgtggcctgg gcgggactgg ggagtggcga gccctcagat 420

cctgcatata agcagctgct ttttgcctgt actgggtctc tctggttaga ccagatctga 480

gcctgggagc tctctggcta actagggaac ccactgctta agcctcaata aagcttgcct 540

tgagtgcttc aagtagtgtg tgcccgtctg ttgtgtgact ctggtaacta gagatccctc 600

agaccctttt agtcagtgtg gaaaatctct agcagtggcg cccgaacagg gacttgaaag 660

cgaaagggaa accagaggag ctctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcagta ttaagcgggg gagaattaga tcgcgatggg 840

aaaaaattcg gttaaggcca gggggaaaga aaaaatataa attaaaacat atagtatggg 900

caagcaggga gctagaacga ttcgcagtta atcctggcct gttagaaaca tcagaaggct 960

gtagacaaat actgggacag ctacaaccat cccttcagac aggatcagaa gaacttagat 1020

cattatataa tacagtagca accctctatt gtgtgcatca aaggatagag ataaaagaca 1080

ccaaggaagc tttagacaag atagaggaag agcaaaacaa aagtaagacc accgcacagc 1140

aagcggccgc tgatcttcag acctggagga ggagatatga gggacaattg gagaagtgaa 1200

ttatataaat ataaagtagt aaaaattgaa ccattaggag tagcacccac caaggcaaag 1260

agaagagtgg tgcagagaga aaaaagagca gtgggaatag gagctttgtt ccttgggttc 1320

ttgggagcag caggaagcac tatgggcgca gcgtcaatga cgctgacggt acaggccaga 1380

caattattgt ctggtatagt gcagcagcag aacaatttgc tgagggctat tgaggcgcaa 1440

cagcatctgt tgcaactcac agtctggggc atcaagcagc tccaggcaag aatcctggct 1500

gtggaaagat acctaaagga tcaacagctc ctggggattt ggggttgctc tggaaaactc 1560

atttgcacca ctgctgtgcc ttggaatgct agttggagta ataaatctct ggaacagatt 1620

tggaatcaca cgacctggat ggagtgggac agagaaatta acaattacac aagcttaata 1680

cactccttaa ttgaagaatc gcaaaaccag caagaaaaga atgaacaaga attattggaa 1740

ttagataaat gggcaagttt gtggaattgg tttaacataa caaattggct gtggtatata 1800

aaattattca taatgatagt aggaggcttg gtaggtttaa gaatagtttt tgctgtactt 1860

tctatagtga atagagttag gcagggatat tcaccattat cgtttcagac ccacctccca 1920

accccgaggg gacccgacag gcccgaagga atagaagaag aaggtggaga gagagacaga 1980

gacagatcca ttcgattagt gaacggatct cgacggtatc gccgaattca caaatggcag 2040

tattcattca caattttaaa agaaaagggg ggattggggg gtacagtgca ggggaaagaa 2100

taatagacat aatagcaaca gatatacaaa ctacagaact acaaaaacaa attataaaaa 2160

ttcaaaattt tcgggtttat tacagagaca gcagagaccc tatttggctg atacgcgtaa 2220

tgaggggatt agagagattt tgttgaatga aaggcagatt gagtcctgct actcgccccc 2280

ttcattcccc ttcattcatg cctcattctt ccgcctccca gccgcctcaa ctggccaaag 2340

ggaagtggag gccctgccac ctgtagggag ggtcccctgg ggcttgccca cagcaaacag 2400

gaagtcacag cctggtgaga tgggcctggg aatcagccac tgagaaagtg ggtctcttgg 2460

gtccctgaat tctttttctg agtccctgca gcagtgaaaa agacacagag gcacatagag 2520

agtgacagag aaagagagag acagagagga gaggcatggg gcagaataag aacagattta 2580

ggagttagaa ctcctgggtt cttttaaaac aatttttctt ttagagacag ggtcttgttg 2640

tgttgcccgg actggagcac agtggctatt cccaggcata atcatggtgc actgcagcct 2700

tgaactcctg ggctcaagcg atccttctac ctcagcctcc caaggacctg ggaccatagg 2760

cgtgtaccac tgtgcctggc ttttgcctgg ttttaaactg aggcagtatg acttgagctc 2820

ttaggcatta attgaagctg tatctcatta actgagggct tatgatgtgc tggacactgg 2880

gctaatagtg ctgaacatat tgtcattttt aatcttcaca aacaatattt gtataggact 2940

gttttctttt cttttttttt tttgaaacag agtctcactc tggtgcccag gctggagtgc 3000

agtggtgtga tctcggctca ctgcaacctc cgcctcctgg tttccagtga ttctcctgcc 3060

tcagcctcct aagtagctgg gattacaggt gtgcgccacc atgcccggct aatttttttt 3120

tttttttttg agaaggagtc tatgtgccca gcattgttct agagcacttg caattagtgg 3180

tgaacaacac ggtctctact ccaaggggct cacattcttg tgcagaaaac agaaatgaac 3240

aaataaacac acaagatcat ttcccgtggt agtgagagct gggatgaaaa taaaacagcg 3300

tggcagggag gaggcaagtg ttgtgagtct ggagggttcc tggagaatgg ggcctgaggc 3360

gtgaccaccg ccttcctctc tggggggact gcctgccgcc cccgcagaca cccatggttg 3420

agtgccctcc aggcccctgc ctgccccagc atcccctgcg cgaagctggg tgccccggag 3480

agtctgacca ccaggatccc tcgagaggga ttcgagctcg gtacctttaa gaccaatgac 3540

ttacaaggca gctgtagatc ttagccactt tttaaaagaa aaggggggac tggaagggct 3600

aattcactcc caacgaagac aagattcgag agatgctgca tataagcagc tgctttttgc 3660

ttgtactggg tctctctggt tagaccagat ctgagcctgg gagctctctg gctaactagg 3720

gaacccactg cttaagcctc aataaagctt gccttgagtg cttcaagtag tgtgtgcccg 3780

tctgttgtgt gactctggta actagagatc cctcagaccc ttttagtcag tgtggaaaat 3840

ctctagc 3847

<210> 8
<211> 3846
<212> DNA
<213> Artificial sequence

<220>
<223> Lentiviral vector containing a promoter derived from the human CD
 19 promoter

<400> 8
tggaagggct aattcactcc caaagaagac aagatatcct tgatctgtgg atctaccaca 60

cacaaggcta cttccctgat tagcagaact acacaccagg gccaggggtc agatatccac 120

tgacctttgg atggtgctac aagctagtac cagttgagcc agataaggta gaagaggcca 180

ataaaggaga gaacaccagc ttgttacacc ctgtgagcct gcatgggatg gatgacccgg 240

agagagaagt gttagagtgg aggtttgaca gccgcctagc atttcatcac gtggcccgag 300

agctgcatcc ggagtacttc aagaactgct gatatcgagc ttgctacaag ggactttccg 360

ctggggactt tccagggagg cgtggcctgg gcgggactgg ggagtggcga gccctcagat 420

cctgcatata agcagctgct ttttgcctgt actgggtctc tctggttaga ccagatctga 480

gcctgggagc tctctggcta actagggaac ccactgctta agcctcaata aagcttgcct 540

tgagtgcttc aagtagtgtg tgcccgtctg ttgtgtgact ctggtaacta gagatccctc 600

agaccctttt agtcagtgtg gaaaatctct agcagtggcg cccgaacagg gacttgaaag 660

cgaaagggaa accagaggag ctctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcagta ttaagcgggg gagaattaga tcgcgatggg 840

aaaaaattcg gttaaggcca gggggaaaga aaaaatataa attaaaacat atagtatggg 900

caagcaggga gctagaacga ttcgcagtta atcctggcct gttagaaaca tcagaaggct 960

gtagacaaat actgggacag ctacaaccat cccttcagac aggatcagaa gaacttagat 1020

cattatataa tacagtagca accctctatt gtgtgcatca aaggatagag ataaaagaca 1080

ccaaggaagc tttagacaag atagaggaag agcaaaacaa aagtaagacc accgcacagc 1140

aagcggccgc tgatcttcag acctggagga ggagatatga gggacaattg gagaagtgaa 1200

ttatataaat ataaagtagt aaaaattgaa ccattaggag tagcacccac caaggcaaag 1260

agaagagtgg tgcagagaga aaaaagagca gtgggaatag gagctttgtt ccttgggttc 1320

ttgggagcag caggaagcac tatgggcgca gcgtcaatga cgctgacggt acaggccaga 1380

caattattgt ctggtatagt gcagcagcag aacaatttgc tgagggctat tgaggcgcaa 1440

cagcatctgt tgcaactcac agtctggggc atcaagcagc tccaggcaag aatcctggct 1500

gtggaaagat acctaaagga tcaacagctc ctggggattt ggggttgctc tggaaaactc 1560

atttgcacca ctgctgtgcc ttggaatgct agttggagta ataaatctct ggaacagatt 1620

tggaatcaca cgacctggat ggagtgggac agagaaatta acaattacac aagcttaata 1680

cactccttaa ttgaagaatc gcaaaaccag caagaaaaga atgaacaaga attattggaa 1740

ttagataaat gggcaagttt gtggaattgg tttaacataa caaattggct gtggtatata 1800

aaattattca taatgatagt aggaggcttg gtaggtttaa gaatagtttt tgctgtactt 1860

tctatagtga atagagttag gcagggatat tcaccattat cgtttcagac ccacctccca 1920

accccgaggg gacccgacag gcccgaagga atagaagaag aaggtggaga gagagacaga 1980

gacagatcca ttcgattagt gaacggatct cgacggtatc gccgaattca caaatggcag 2040

tattcattca caattttaaa agaaaagggg ggattggggg gtacagtgca ggggaaagaa 2100

taatagacat aatagcaaca gatatacaaa ctacagaact acaaaaacaa attataaaaa 2160

ttcaaaattt tcgggtttat tacagagaca gcagagaccc tatttggctg atacgcgtaa 2220

tgaggggatt agagagattt tgttgaatga aaggcagatt gagtcctgct actcgccccc 2280

ttcattcccc ttcattcatg cctcattctt ccgcctccca gccgcctcaa ctggccaaag 2340

ggaagtggag gccctgccac ctgtagggag ggtcccctgg ggcttgccca cagcaaacag 2400

gaagtcacag cctggtgaga tgggcctggg aatcagccac tgagaaagtg ggtctcttgg 2460

gtccctgaat tctttttctg agtccctgca gcagtgaaaa agacacagag gcacatagag 2520

agtgacagag aaagagagag acagagagga gaggcatggg gcagaataag aacagattta 2580

ggagttagaa ctcctgggtt cttttaaaac aatttttctt ttagagacag ggtcttgttg 2640

tgttgcccgg actggagcac agtggctatt cccaggcata atcatggtgc actgcagcct 2700

tgaactcctg ggctcaagcg atccttctac ctcagcctcc caaggacctg ggaccatagg 2760

cgtgtaccac tgtgcctggc ttttgcctgg ttttaaactg aggcagtatg acttgagctc 2820

ttaggcatta attgaagctg tatctcatta actgagggct tatgatgtgc tggacactgg 2880

gctaatagtg ctgaacatat tgtcattttt aatcttcaca aacaatattt gtataggact 2940

gttttctttt cttttttttt ttgaaacaga gtctcactct ggtgcccagg ctggagtgca 3000

gtggtgtgat ctcggctcac tgcaacctcc gcctcctggt ttccagtgat tctcctgcct 3060

cagcctccta agtagctggg attacaggtg tgcgccacca tgcccggcta attttttttt 3120

tttttttgga gaaggagtct atgtgcccag cattgttcta gagcacttgc aattagtggt 3180

gaacaacacg gtctctactc caaggggctc acattcttgt gcagaaaaca gaaatgaaca 3240

aataaacaca caagatcatt tcccgtggta gtgagagctg ggatgaaaat aaaacagcgt 3300

ggcagggagg aggcaagtgt tgtgagtctg gagggttcct ggagaatggg gcctgaggcg 3360

tgaccaccgc cttcctctct ggggggactg cctgccgccc ccgcagacac ccatggttga 3420

gtgccctcca ggcccctgcc tgccccagca tcccctgcgc gaagctgggt gccccggaga 3480

gtctgaccac caggatccct cgagagggat tcgagctcgg tacctttaag accaatgact 3540

tacaaggcag ctgtagatct tagccacttt ttaaaagaaa aggggggact ggaagggcta 3600

attcactccc aacgaagaca agattcgaga gatgctgcat ataagcagct gctttttgct 3660

tgtactgggt ctctctggtt agaccagatc tgagcctggg agctctctgg ctaactaggg 3720

aacccactgc ttaagcctca ataaagcttg ccttgagtgc ttcaagtagt gtgtgcccgt 3780

ctgttgtgtg actctggtaa ctagagatcc ctcagaccct tttagtcagt gtggaaaatc 3840

tctagc 3846

<210> 9
<211> 24
<212> DNA
<213> Artificial sequence

<220>
<223> PCR primer

<400> 9
gttacgcgtc ccgtggtagt gaga 24

<210> 10
<211> 24
<212> DNA
<213> Artificial sequence

<220>
<223>  PCR primer

<400>  10
ttgggatccg gtggtcagac tctc                                              24


<210>  11
<211>  27
<212>  DNA
<213>  Artificial sequence

<220>
<223>  PCR primer

<400>  11
gttacgcgta atgaggggat tagagag                                           27


<210>  12
<211>  26
<212>  DNA
<213>  Artificial sequence

<220>
<223>  PCR primer

<400>  12
ttgggatccg gtggtcagac tctccg                                            26


<210>  13
<211>  36
<212>  DNA
<213>  Artificial sequence

<220>
<223>  PCR primer

<400>  13
gctctcacta ccacgggacc actttctcag tggctg                                 36


<210>  14
<211>  36
<212>  DNA
<213>  Artificial sequence

<220>
<223>  PCR primer

<400>  14
cagccactga gaaagtggtc ccgtggtagt gagagc                                 36


<210>  15
<211>  20
<212>  DNA
<213>  Artificial sequence

<220>
<223>  PCR primer

<400>  15

agcaaagacc ccaacgagaa                                                20

<210>  16
<211>  16
<212>  DNA
<213>  Artificial sequence

<220>
<223>  PCR primer

<400>  16
ggcggcggtc acgaac                                                    16

<210>  17
<211>  22
<212>  DNA
<213>  Artificial sequence

<220>
<223>  Detection probe

<400>  17
cgcgatcaca tggtcctgct gg                                             22

<210>  18
<211>  21
<212>  DNA
<213>  Artificial sequence

<220>
<223>  PCR primer

<400>  18
aagttcatct gcaccaccgg c                                              21

<210>  19
<211>  21
<212>  DNA
<213>  Artificial sequence

<220>
<223>  PCR primer

<400>  19
aagaagatgg tgcgctcctg g                                              21

<210>  20
<211>  20
<212>  DNA
<213>  Artificial sequence

<220>
<223>  PCR primer

<400>  20
ccgccatctg atgagcagtt                                                20

<210>  21
<211>  20

<212>  DNA
<213>  Artificial sequence

<220>
<223>  PCR primer

<400>  21
tgaagctctt tgtgacgggc                                                    20